# EUROPEAN PATENT SPECIFICATION

(11) **EP 2 510 091 B1**
(45) Date of publication and mention of the grant of the patent: **28.06.2017**
(21) Application number: 10801023.2
(22) Date of filing: 07.12.2010
(51) Int. Cl.: C12N 9/16, C12N 15/861, A61K 48/00, A61K 38/00

(54) **METHOD AND COMPOSITION TO INCREASE RADIATION-INDUCED TUMOR THERAPEUTIC EFFECTS**
VERFAHREN UND ZUSAMMENSETZUNG ZUR ERHÖHUNG DER WIRKUNG VON STRAHLUNGSINDUZIERTEN TUMORTHERAPEUTIKA
PROCÉDÉ ET COMPOSITION POUR AUGMENTER LES EFFETS THÉRAPEUTIQUES ANTICANCÉREUX INDUITS PAR RAYONNEMENT

(30) Priority: 08.12.2009 US 283696 P
(43) Date of publication of application: 17.10.2012
(73) Proprietor: Memorial Sloan-Kettering Cancer Center, New York, NY 10065 (US); Vascular Biogenics Ltd., 60376 Or Yehuda (IL)
(72) Inventor: KOLESNICK, Richard, N., New York, NY 10022 (US); STANCEVIC, Branka, New York, NY 10075 (US); SADELAIN, Michel, New York, NY 10025 (US); FUKS, Zvi, New York NY 10028 (US); VARDA-BLOOM, Nira, 45309 Hod-hasharon (IL); HARATS, Dror, 52648 Ramat Gan (IL)
(74) Representative: Green, Mark Charles
(86) International application number: PCT/US2010/059204
(87) International publication number: WO 2011/071859

(56) References cited:
- WO-A1-96/14061
- VARDA-BLOOM NITA ET AL: "Genetic manipulation of bone marrow-derived endothelial progenitors to modulate sphingomyelinase-mediated endothelial cell apoptosis within tumors." PROCEEDINGS OF THE AMERICAN ASSOCIATION FOR CANCER RESEARCH ANNUAL MEETING, vol. 46, April 2005 (2005-04), XP002626077 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- GARCIA-BARROS MONICA ET AL: "Tumor response to radiotherapy regulated by endothelial cell apoptosis." SCIENCE (NEW YORK, N.Y.), vol. 300, no. 5622, 16 May 2003 (2003-05-16), pages 1155-1159, XP002626078 ISSN: 1095-9203 cited in the application
- SMITH ERIC L ET AL: "Acid sphingomyelinase overexpression enhances the antineoplastic effects of irradiation in vitro and in vivo." MOLECULAR THERAPY : THE JOURNAL OF THE AMERICAN SOCIETY OF GENE THERAPY, vol. 16, no. 9, September 2008 (2008-09), pages 1565-1571, XP002626079 ISSN: 1525-0024
- Stancevic, Branka: "Ceramide-rich platforms regulate ionizing radiation sensitivity in endothelium"[Online] 3407232, 1 November 2010 (2010-11-01), XP002626080 Dissertation for the Degree of Doctor of Philosophy, Cornell University Retrieved from the Internet: URL:http://gradworks.umi.com/34/07/3407232 .html> [retrieved on 2011-03-02]
- WAEHLER REINHARD ET AL: "Engineering targeted viral vectors for gene therapy", NATURE REVIEWS GENETICS, NATURE PUBLISHING GROUP, GB, vol. 8, no. 8, 1 August 2007 (2007-08-01), pages 573-587, XP002663591, ISSN: 1471-0056, DOI: 10.1038/NRG2141 [retrieved on 2007-07-03]
- EL-ANEED ANAS: "An overview of current delivery systems in cancer gene therapy.", JOURNAL OF CONTROLLED RELEASE, vol. 94, no. 1, 8 January 2004 (2004-01-08), pages 1-14, ISSN: 0168-3659

## Description

### FIELD OF THE INVENTION

The present invention is directed to a novel method for radiotherapy of solid tumors.

### BACKGROUND OF THE INVENTION

Conventional cancer therapies (chemotherapy, surgery, radiation) produce a high rate of early stage disease regression, but many cancers reoccur. Additionally, in advanced stages of cancer, many patients ultimately die. As a single modality, there are certain limitations to each of these therapies which include, for example, the dose of chemotherapeutic drugs, the extent of surgical resection possible, or the dose of radiation and volume to be irradiated. Improved results are often achieved when these modalities are used in combination. For example, surgical resection preceded or followed by chemotherapy has proven effective in some cancers. The utility of radiation therapy can be limited for a number of reasons, including dose limitation to avoid damage to non-cancerous tissue in the radiation field and the development of radiation resistance. There is, therefore, a need to develop a therapy that can prevent unwanted effects on healthy cells while achieving the desired effect on cancer cells and that can avoid the development of resistance mechanisms that allow cancer cells to evade the effects of therapy.

Ionizing radiation has long been used as a therapy for solid tumors. Until recently, it was believed that ionizing radiation acted exclusively on tumor cells to induce cellular DNA damage and mitotic cell death. However, genetic and pharmacologic studies indicate that tumor response to radiation is determined not only by the inherent radiosensitivity of the tumor cells themselves but also by the radiosensitivity of the tumor microvasculature.

Acid sphingomyelinase (ASMase) catalyzes the cleavage of sphingomyelin to generate ceramide, which has been shown to act as a 'second messenger' in cell signaling pathways. Various stimuli, including ionizing radiation, result in the activation of ASMase and its translocation to the outer leaflet of the cell membrane. Stimulus-induced activation and translocation of ASMase to the sphingomyelin-rich outer leaflet of the cell membrane leads, in turn, to the generation of ceramide from sphingomyelin. The unique biophysical properties of ceramide mediate membrane reorganization, lateral movement of lipids and formation of ceramide-rich platforms (CRPs). CRPs, in turn, concentrate receptors and effector molecules, leading to signal amplification and transduction that triggers apoptosis.

Cells derived from individuals with Neimann-Pick disease, an inherited deficiency of ASMase, fail to generate ceramide in response to cellular stressors and are resistant to stress-induced apoptosis. ASMase-deficient mice are, similarly, 'protected' from apoptotic cell death, including that mediated by ionizing radiation, activated cytotoxic T lymphocytes and by cytokines.

Comparison of the growth and response to ionizing radiation of tumors implanted in wild-type and ASMase-deficient mice demonstrates that ASMase and (host-derived) tumor vasculature play an important role, not only in tumor growth, but in tumor response to radiotherapy. MCA/129 fibrosarcomas and B16F1 melanomas grown in apoptosis-resistant ASMase-deficient mice display markedly reduced baseline microvascular endothelial apoptosis and grow 200% to 400% faster than tumors grown in wild-type mice. Moreover, tumors grown in ASMase-deficient mice exhibit reduced endothelial apoptosis in response to radiation and, unlike tumors grown in wild-type mice, are resistant to single-dose radiotherapy at all radiation doses tested. Thus, ASMase-mediated microvascular apoptosis regulates tumor growth and regulates tumor response to radiation at clinically relevant dosage ranges.

### SUMMARY OF THE INVENTION

This present disclosure is drawn to methods and compositions for treating cancer. In some embodiments, at least a fragment of a gene can be provided to alter the levels of a protein. The gene can be delivered to the cell(s) of interest using a vector (viral or non-viral), and expression of the protein can result in a therapeutic effect. Fragments of one or more genes can also be delivered by the vector (or also multiple vectors each encoding at least a portion of one gene). In one embodiment, the methods used for altering gene expression can be used to treat cancer, or other proliferative diseases. In other embodiments, features that target the vector are included. In further embodiments, the composition and method employs a promoter/enhancer specific to angiogenic endothelium to increase levels of ASMase in tumor neovasculature. Additional features can be added to the vector for enhancement of its therapeutic efficacy or to improve safety. Administration can be accomplished by any number of methods known to a skilled artisan including, without limitation, intravenous injection or infusion, oral administration, treatment *ex vivo,* local injection, or transfection or transduction. Such administration may include administration alone or in combination with other carriers, adjuvants, diluents, or pharmaceutically active ingredients.

Although endothelial cells synthesize 20 times as much ASMase as any other cell type investigated in the body, mostly in a non-lysosomal secretory form, increased levels of ASMase sensitize endothelial cells to radiation-induced apoptosis and thereby enhance tumor response to radiotherapy. By increasing the effectiveness of radiotherapy without risking damage to normal tissue, the disclosed methods and compositions expand the benefit of radiotherapy to solid tumors resistant to conventional fractionated-dose radiotherapy and render single-dose radiotherapy approaches feasible with lower doses of radiation.

Disclosed herein is an expression vector as claimed in Claim 1. In another embodiment, the angiogenic endothelium-specific transcriptional regulatory sequences are selected from the group consisting of promoters and enhancers.

In another embodiment, the promoter is pre-proendothelin-1 promoter or modifications thereof. In another embodiment, the promoter is PPE-1 (x3). In another embodiment, the enhancer is HIF2a-Ets-1 enhancer.

In one embodiment disclosed herein, the expression vector of the inventionis used in a method to treat cancer by increasing radiation-induced damage to a tumor without increasing radiation-induced side effects is provided comprising increasing secretory ASMase levels specifically in tumor endothelium, and inducing apoptosis of tumor endothelial cells by treating the tumor with radiation.

In another embodiment, the cancer is a solid tumor. In another embodiment, the increase in radiation-induced damage to cancer without an increase in radiation-induced side effects is achieved by sensitizing the tumor to radiation. In another embodiment, the increase in radiation-induced damage to cancer without an increase in radiation-induced side effects is achieved by sensitizing the angiogenic epithelium of the tumor to radiation

In yet another embodiment, secretory ASMase levels are increased specifically in tumor endothelium through the administration of a gene therapy construct. In one embodiment, the gene therapy construct is the construct comprising a region coding for a functional secretory ASMase linked to transcriptional regulatory sequences that confer tissue-specific expression of the secretory ASMase

In another embodiment, ceramide levels are increased specifically in tumor endothelium through the administration of the gene therapy construct.

### DESCRIPTION OF FIGURES

This patent or application file contains at least one drawing executed in color. Copies of this patent or patent application publication with color drawing(s) will be provided by the Office upon request and payment of the necessary fee.

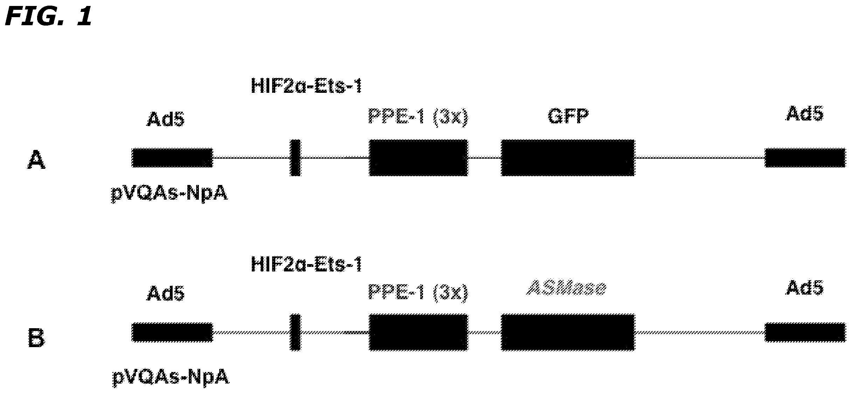

FIG. 1 depicts a schematic diagram of the disclosed endothelial-specific adenoviral vectors. The constructs were designed to target gene expression specifically to endothelium and to further enhance target gene expression under hypoxic conditions, such as those characteristic of tumors. Each cassette contains a hypoxia-inducible enhancer, HIF2α-Ets-1 (132 bp), and a modified murine pre-proendothelin promoter, PPE-1(3x) (1.5 kb), controlling the expression of green fluorescent protein (GFP) (2.25 kb) (FIG. 1A) or ASMase (3.55 kb) (FIG. 1B). Cassettes containing the enhancer, the promoter and the targeted gene were inserted within a replication-defective adenovirus serotype 5 genome to generate Ad5HEPPE-3x(GFP) or Ad5HEPPE-3x(ASM).

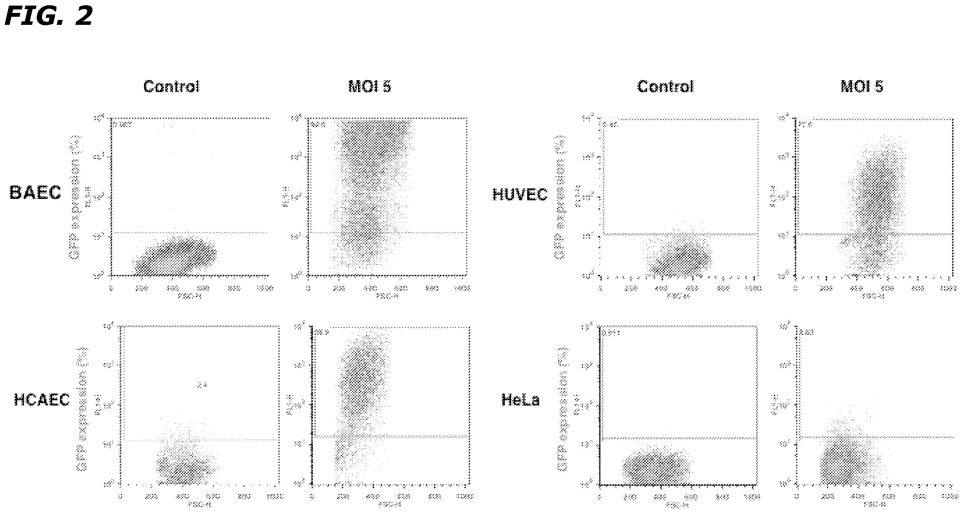

FIG. 2 depicts induction of GFP expression in endothelial cells after infection with Ad5HEPPE-3x(GFP). Endothelial cells (BAEC, HUVEC and HCAEC) and non-endothelial cells (HeLa) were infected with Ad5HEPPE-3x(GFP). GFP expression was measured in live cells following detachment 72 hours post-infection (multiplicity of infection [MOI] = 5) by flow cytometry. Data are representative of three independent experiments.

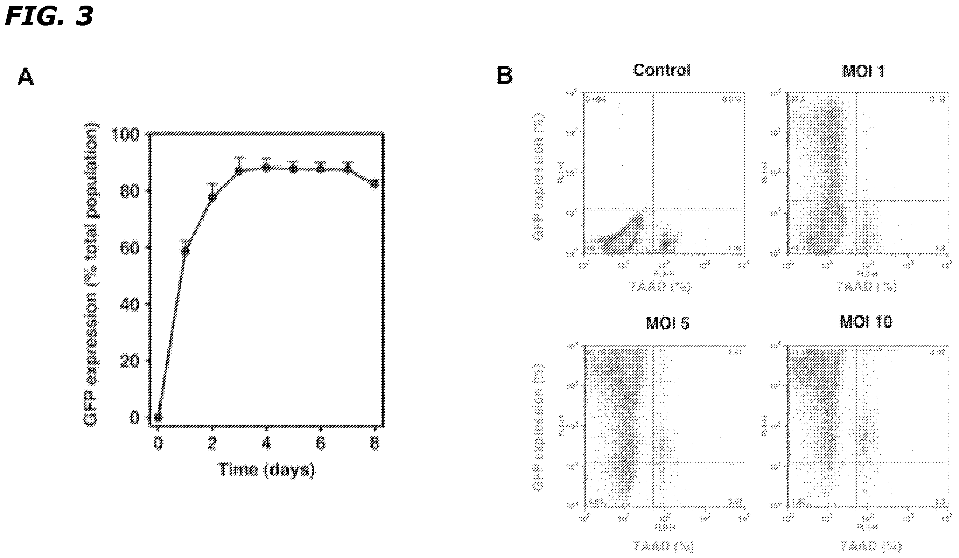

FIG. 3 depicts that infection with Ad5HEPPE-3x(GFP) yields maximal GFP expression for at least 7 days and does not induce toxicity. BAEC were infected with Ad5HEPPE-3x(GFP) at MOI of 1 (FIGs. 3A and 3B), 5 (FIG. 3B), and 10 (FIG. 3B). GFP expression was measured in live cells at various time points post-infection by flow cytometry (FIG. 3A). Data (mean ± SE) are collated from three independent experiments. Viability was measured in non-permeabilized cells by flow cytometry by incorporation of 7AAD (FIG. 3B). Non-viable cells were identified as 7AAD-positive (upper and bottom right quadrants) by Flow Jo analysis. Data are representative of three independent experiments.

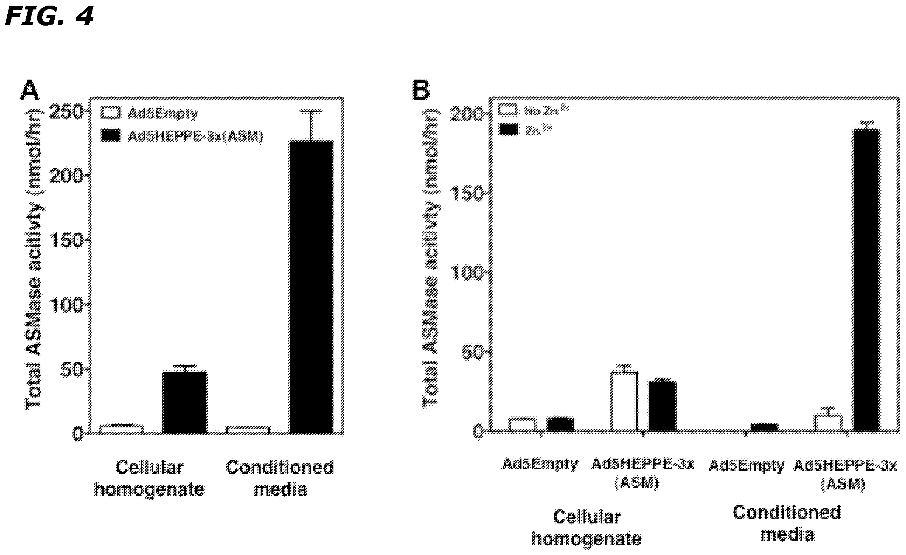

FIG. 4 depicts that overexpression of ASMase leads to an increase in the activity of both lysosomal, Zn²⁺-independent, and secretory, Zn²⁺-dependent ASMase. Cellular homogenates and serum-free conditioned media were harvested from BAEC infected with Ad5Empty or Ad5HEPPE-3x(ASM) and assayed for ASMase activity. ASMase activity was determined at pH 5.0 using [¹⁴C]sphingomyelin as a substrate in the presence of 1 mM EDTA (cellular homogenates) or 0.1 mM Zn²⁺ (conditioned media) (FIG. 4A). Dependence of the ASMase activity on extracellular addition of Zn²⁺ was determined by assaying activity in cellular homogenates and conditioned media in the presence of 1 mM EDTA (white bars) or 1 mM Zn²⁺ (black bars) (FIG. 4B). Data (mean ± SE) are collated from three independent experiments performed in triplicate.

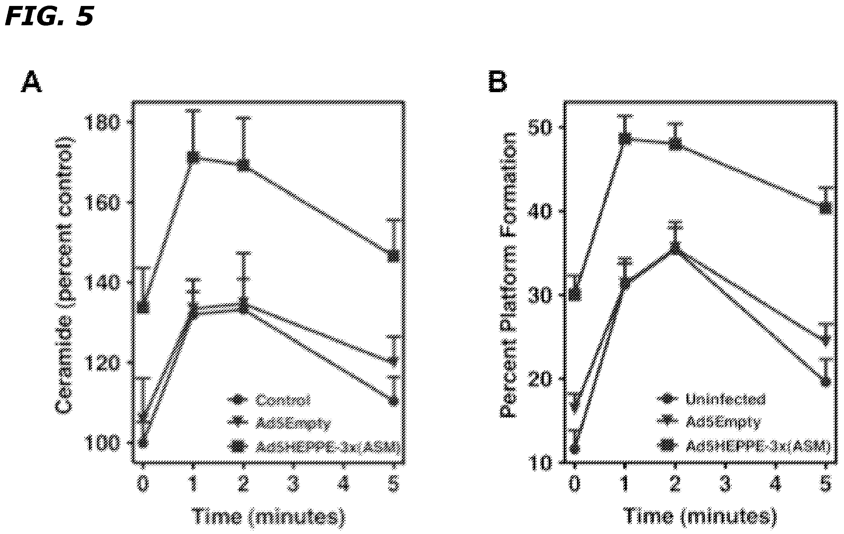

FIG. 5 depicts that overexpression of ASMase leads to a baseline and ionizing irradiation (IR)-induced increase in ceramide generation and platform formation. BAECs, untreated or infected with Ad5Empty or Ad5HEPPE-3x(ASM), were stimulated with 10 Gy irradiation and incubated at 37°C for the indicated times. Ceramide content was measured using the diacylglycerol (DG) kinase assay (FIG. 5A). Data (mean ± SE) are collated from two independent experiments performed in triplicate. Cells containing CRPs, defined as cells containing concentration of fluorescence into less than 25% of the cell surface, were identified by standard fluorescent microscopy following staining with Texas Red-labeled anti-ceramide antibody (FIG. 5B). Data (mean ± 95% CI) are collated from three experiments in which 200 cells were analyzed per point.

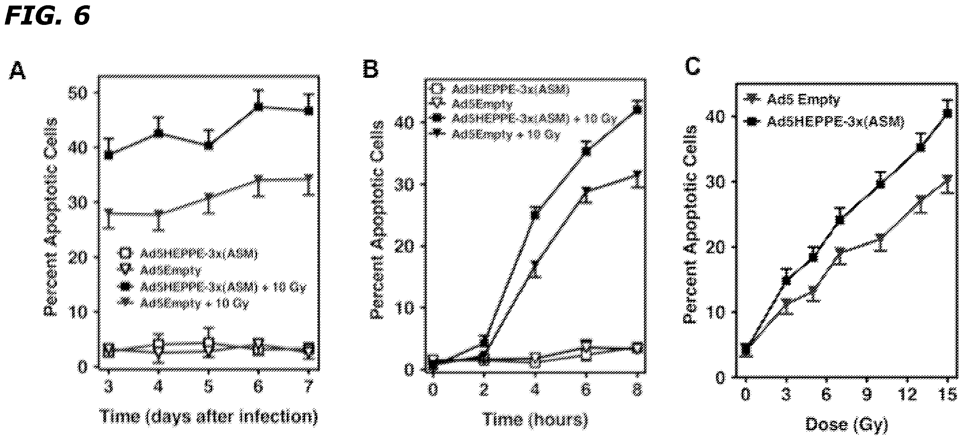

FIG. 6 depicts that increase in ASMase activity and ceramide generation radiosensitizes BAEC in a time- and dose-dependent manner. BAEC infected with Ad5Empty or Ad5HEPPE-3x(ASM) were left untreated or stimulated with 10 Gy irradiation at indicated times after the infection (FIG. 6A) or three days after the infection (FIG. 6B). Apoptosis was assessed at 8 hours after stimulation (FIG. 6A) or at various time points after stimulation (FIG. 6B) by bisbenzimide staining. BAECs infected with Ad5Empty or Ad5HEPPE-3x(ASM) were stimulated with various doses of irradiation, and apoptosis was assessed by bisbenzimide staining 8 hours after stimulation (FIG. 6C). Data (mean ± SE) are collated from three experiments performed in triplicate in which 400 nuclei were analyzed per point.

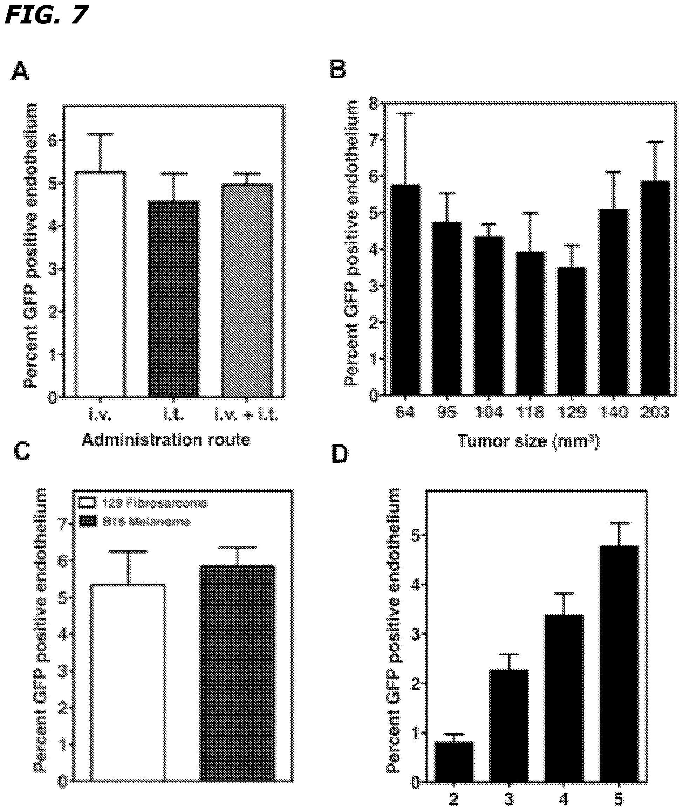

FIG. 7 depicts optimization of the adenovirus administration. 1x10¹⁰ PFU of Ad5HEPPE-3x(GFP) was administered to mice bearing B16F1 melanoma (FIG. 7A and FIG. 7B) or MCA/129 fibrosarcoma (FIG. 7C and FIG. 7D) by intravenous (FIGs. 7 A-D) or intratumoral (FIG. 7A) injection. Tumors were excised 2-5 (FIG. 7D) or 5 (FIGs. 7 A-D) days post administration of virus, and reporter gene expression was assessed following immunostaining of tumor sections with GFP and MECA-32. Data (mean ± SE) represent GFP-positive endothelial cells collated from 20 fields from one or two similar experiments employing at least two animals per group.

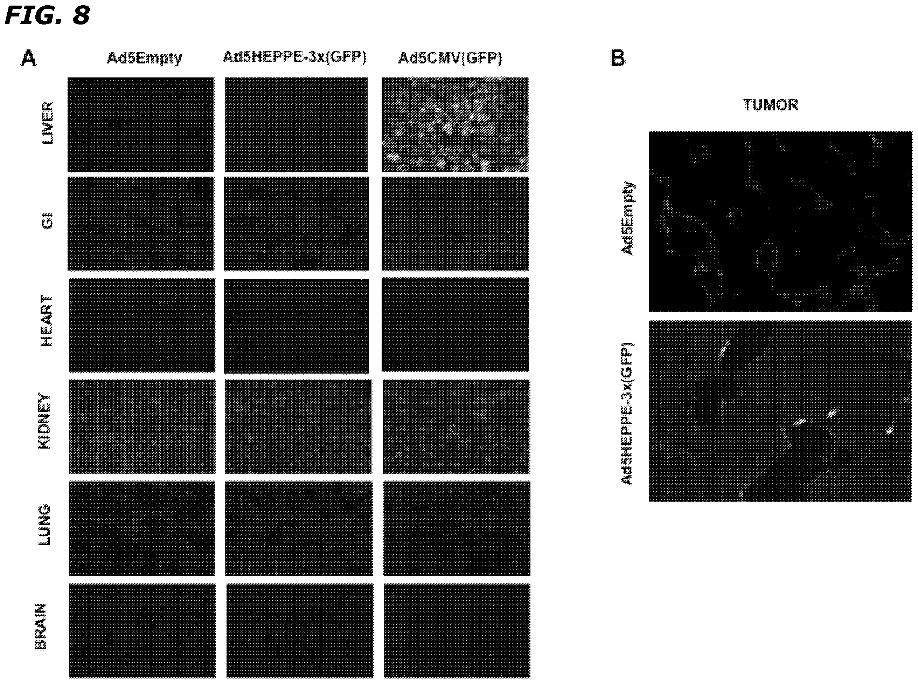

FIG. 8 depicts that intravenous administration of Ad5HEPPE-3x(GFP) results in selective expression of GFP in tumor endothelium. 1x10¹⁰ PFU of Ad5Empty, Ad5HEPPE-3x(GFP) or Ad5CMV(GFP) was intravenously administered to MCA/129 fibrosarcoma-bearing mice. Five days post administration of virus, normal (FIG. 8A) and tumor (FIG. 8B) tissues were excised and GFP expression was visualized by standard fluorescent microscopy following staining of tissue sections with anti-GFP (green) and anti-MECA-32 (red) antibodies. Twenty fields per sample were analyzed. Representative images are shown at 20x magnification.

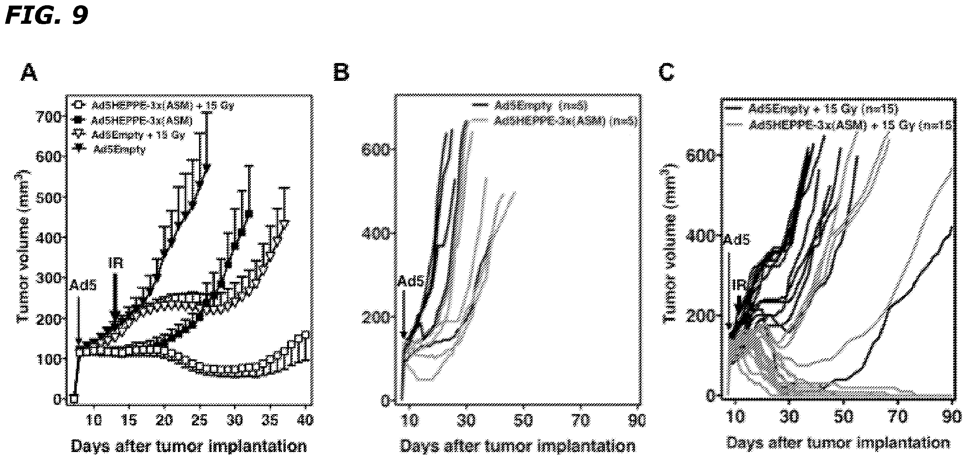

FIG. 9 depicts that expression of ASMase in tumor endothelium of asmase^{-/-}mice restores sensitivity of MCA/129 fibrosarcoma to radiation. 1x10¹⁰ PFU of Ad5Empty or Ad5HEPPE-3x(ASM) was intravenously administered to asmase^{-/-} mice bearing MCA/129 fibrosarcoma. Five days post administration of virus, tumors were locally irradiated with 15 Gy (FIGs. 9A and C) or left untreated (FIGs. 9A and B). Response of MCA/129 fibrosarcoma to Ad5HEPPE-3x(ASM) and to single-dose radiotherapy presented as tumor volume (FIG. 9A). Data (mean ± SE) are collated from 5 (0 Gy) and 15 (15 Gy) animals per group. Response of MCA/129 fibrosarcoma to Ad5HEPPE-3x(ASM) (FIGs. 9B and C) and single-dose radiotherapy (FIG. 9C) presented as tumor volume. Tumors were measured daily for 40 days and twice weekly thereafter.

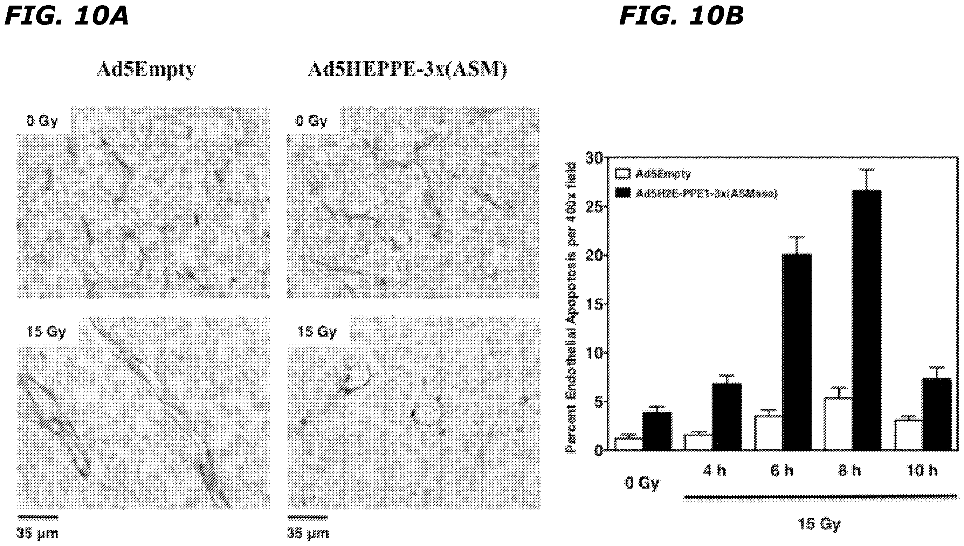

FIG. 10 depicts that expression of ASMase in tumor endothelium of asmase^{-/-}mice leads to radiation-induced endothelial apoptosis. 1x10¹⁰ PFU of Ad5Empty or Ad5HEPPE-3x(ASM) was intravenously administered to asmase^{-/-} mice bearing MCA/129 fibrosarcoma. Five days post administration of virus, tumors were locally irradiated with 15 Gy or left untreated. Tumor samples were obtained before or 4, 6, 8 or 10 hours following irradiation, fixed in paraformaldehyde, and embedded in paraffin blocks. Tissue sections were stained with an endothelial specific (anti-MECA-32, blue) and TUNEL (brown) antibodies. Representative cross sections of MCA/129 fibrosarcoma from animals treated with Ad5Empty or Ad5HEPPE-3x(ASM) and 15 Gy excised 6 hours post radiation (FIG. 10A). Quantification of the effect of administration of virus on radiation-induced endothelial cell apoptosis at 4, 6, 8 and 10 hours post irradiation (FIG. 10B). Data (mean ± SE) represent TUNEL-positive endothelial cells quantified from twenty 400x magnification fields from an experiment employing two animals per group.

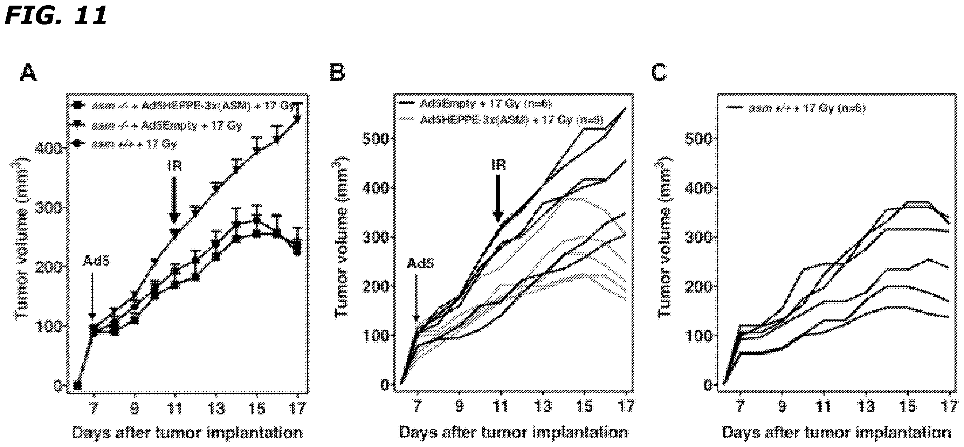

FIG. 11 depicts that regulation of Ad5HEPPE-3x(ASM)-mediated tumor response to radiation is not dependent on the host immune response. 1x10¹⁰ PFU of Ad5Empty or Ad5HEPPE-3x(ASM) was intravenously administered to SCID^{-/-}asmase^{-/-}bearing MCA/129 fibrosarcoma. SCID^{-/-}asmase^{+/+} mice bearing MCA/129 fibrosarcoma were left untreated. Four days post administration of virus, (i.e., 11 days after tumor implantation in SCID^{-/-}asmase^{+/+} mice), tumors were locally irradiated with 17 Gy. Response of MCA/129 fibrosarcoma in SCID^{-/-}asmase^{-/-} mice to single-dose radiotherapy plus Ad5HEPPE-3x (ASM) or Ad5Empty and of SCID^{-/-}asmase^{+/+} mice to single-dose radiotherapy presented as tumor volume (FIG. 11A). Data (mean ± SE) are collated from five animals per group. Response of MCA/129 fibrosarcoma to Ad5Empty or Ad5HEPPE-3x(ASM) (FIG. 11 B) and single-dose radiotherapy (FIGs. 11B and C) presented as tumor volume. Tumors were measured daily.

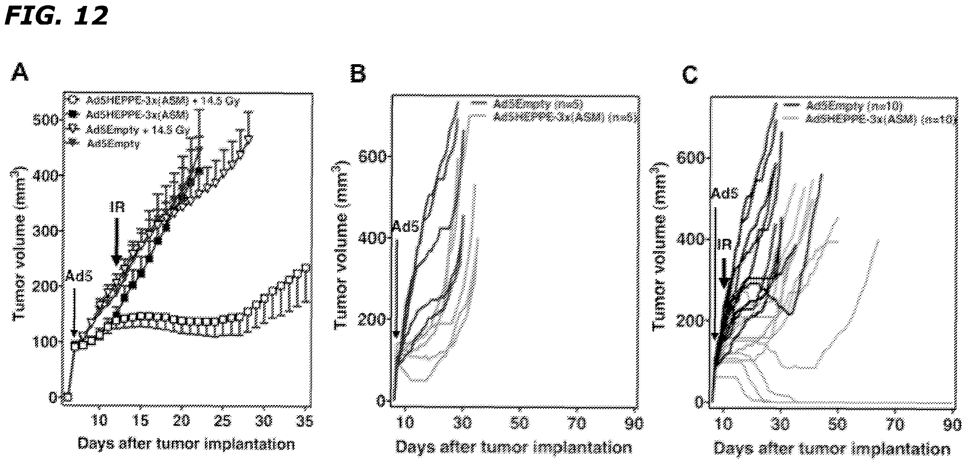

FIG. 12 depicts that overexpression of ASMase in tumor endothelium radiosensitizes MCA/129 fibrosarcoma to 14.5 Gy. 1x10¹⁰ PFU of Ad5Empty or Ad5HEPPE-3x(ASM) was intravenously administered to SV129/C57^{asm+/+JAX} mice bearing MCA/129 fibrosarcoma. Five days post administration of virus, tumors were locally irradiated with 14.5 Gy (FIGs. 12A and C) or left untreated (FIGs. 12A and B). Response of MCA/129 fibrosarcoma to Ad5HEPPE-3x(ASM) and single-dose radiotherapy presented as tumor volume (FIG. 12A). Data (mean ± SE) are collated from five (0 Gy) and ten (14.5 Gy) animals per group. Response of MCA/129 fibrosarcoma to Ad5HEPPE-3x(ASM) (FIGs. 12B and C) and single-dose radiotherapy (FIG. 12C) presented as tumor volume. Tumors were measured daily for 40 days and twice weekly thereafter.

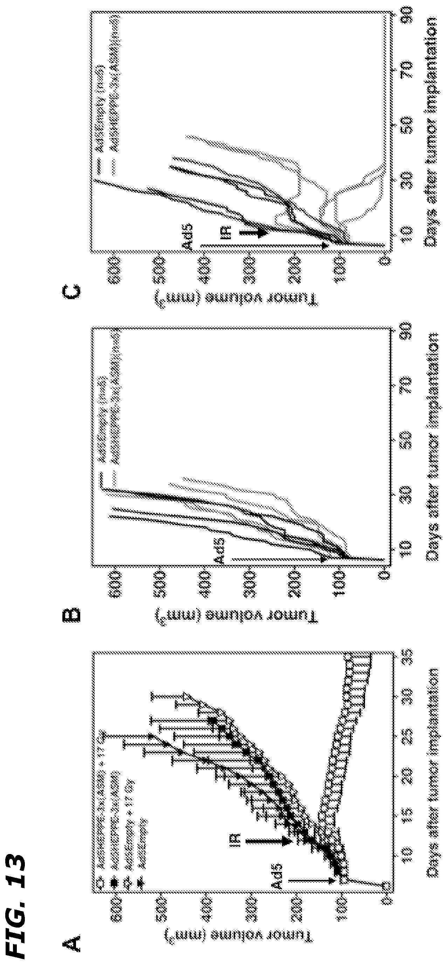

FIG. 13 depicts that overexpression of ASMase in tumor endothelium radiosensitizes MCA/129 fibrosarcoma to 17 Gy. 1x10¹⁰ PFU of Ad5Empty or Ad5HEPPE-3x(ASM) was intravenously administered to SV129/C57^{asm+/+JAX} mice bearing MCA/129 fibrosarcoma. Five days post administration of virus, tumors were locally irradiated with 17 Gy (FIGs. 13A and C) or left untreated (FIGs. 13A and B). Response of MCA/129 fibrosarcoma to Ad5HEPPE-3x(ASM) and single-dose radiotherapy presented as tumor volume (FIG. 13A). Data (mean ± SE) are collated from five animals per group. Response of MCA/129 fibrosarcoma to Ad5HEPPE-3x(ASM) (FIGs. 13B and C) and single-dose radiotherapy (FIG. 13C) presented as tumor volume. Tumors were measured daily for 40 days and twice weekly thereafter.

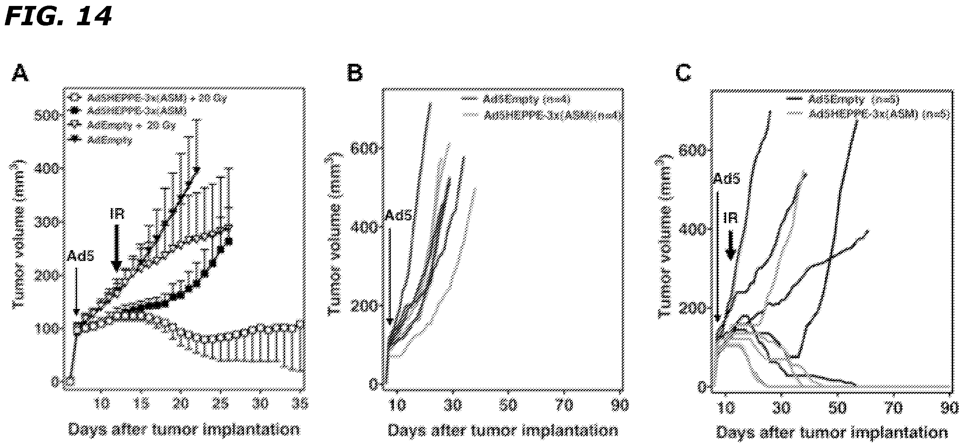

FIG. 14 depicts that overexpression of ASMase in tumor endothelium radiosensitizes MCA/129 fibrosarcoma to 20 Gy. 1x10¹⁰ PFU of Ad5Empty or Ad5HEPPE-3x(ASM) was intravenously administered to SV129/C57^{asm+/+JAX} mice bearing MCA/129 fibrosarcoma. Five days post administration of virus, tumors were locally irradiated with 20 Gy (FIGs. 14A and C) or left untreated (FIGs. 14A and B). Response of MCA/129 fibrosarcoma to Ad5HEPPE-3x(ASM) and single-dose radiotherapy presented as tumor volume (FIG. 14A). Data (mean ± SE) are collated from four (0 Gy) and five (20 Gy) animals per group. Response of MCA/129 fibrosarcoma to Ad5HEPPE-3x(ASM) (FIGs. 14B and C) and single-dose radiotherapy (FIG. 14C) presented as tumor volume. Tumors were measured daily for 40 days and twice weekly thereafter.

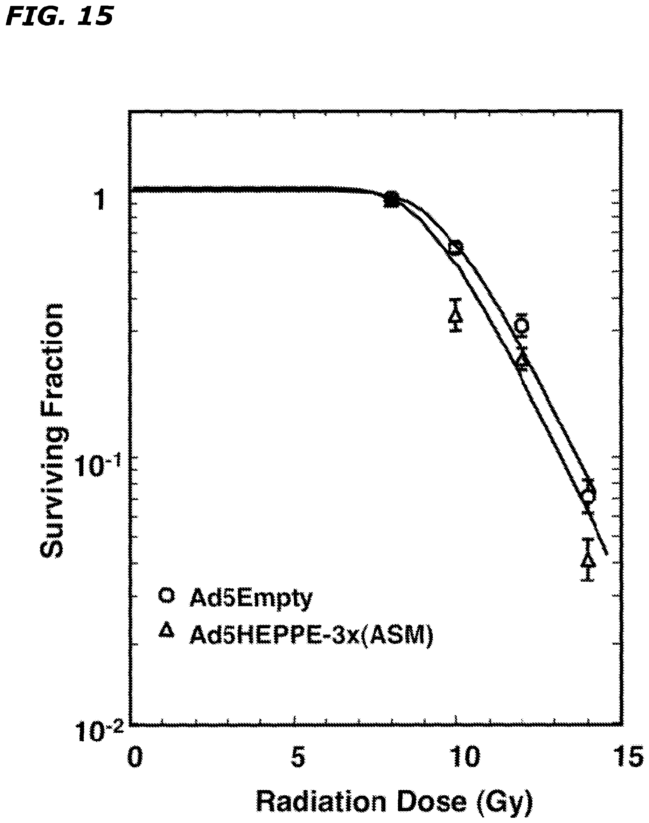

FIG. 15 depicts that overexpression of ASMase in angiogenic endothelium does not radiosensitize the gastrointestinal (GI) tract. 1x10¹⁰ PFU of Ad5Empty or Ad5HEPPE-3x(ASM) was intravenously administered to SV129/C57^{asm+/+JAX} mice. Five days post administration of virus, mice were administered total body irradiation at doses of 8, 10, 12 and 15 Gy or left untreated. Full transverse sections of proximal jejunum were obtained 3.5 days post irradiation, and crypt survival was assessed by the crypt microcolony assay. Data from computation of the surviving fractions at each dose level were compiled from two concomitantly irradiated animals, with 10-20 circumferences scored per mouse. Surviving fraction per dose was calculated with the FIT software program. Data are represented as mean ± SE.

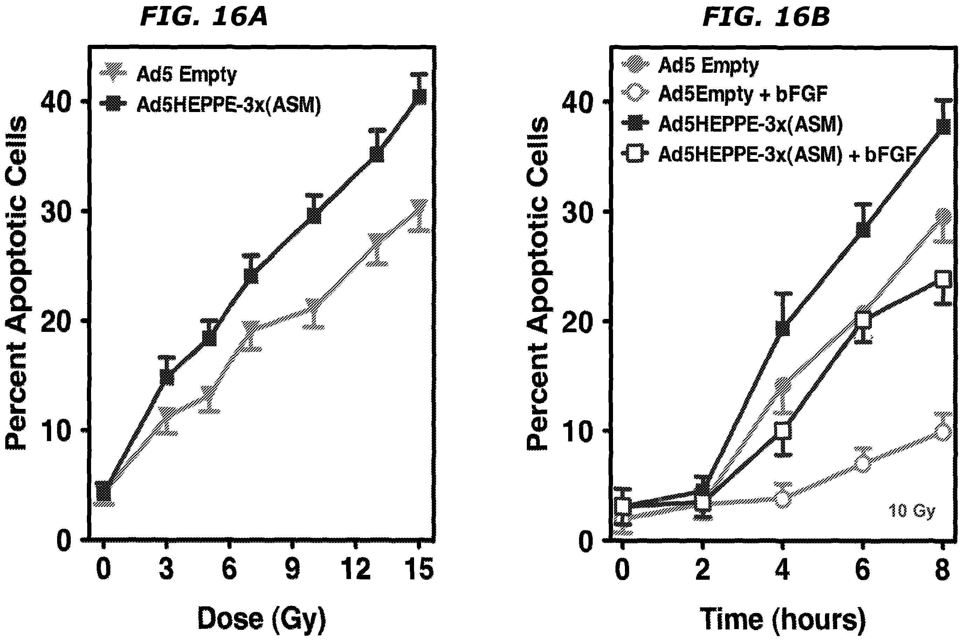

FIG. 16 depicts that overexpression of ASMase via Ad5HEPPE-3x(ASM) leads to radiosensitization of BAEC and attenuates bFGF protective effect from IR-induced apoptosis. BAEC transduced with Ad5Empty or Ad5HEPPE-3x(ASM) were treated with various doses of IR (FIG. 16A) or pretreated with 1ng/ml bFGF 15 minutes prior to stimulation with 10 Gy IR (FIG. 16B). Apoptosis was assessed at 8 hours (FIG. 16A) or at various time points after IR (FIG. 16B) by morphology analysis following bisbenzimide staining. Data (mean ± SE) were collated from 3 experiments performed in triplicate in which 400 nuclei were analyzed per sample.

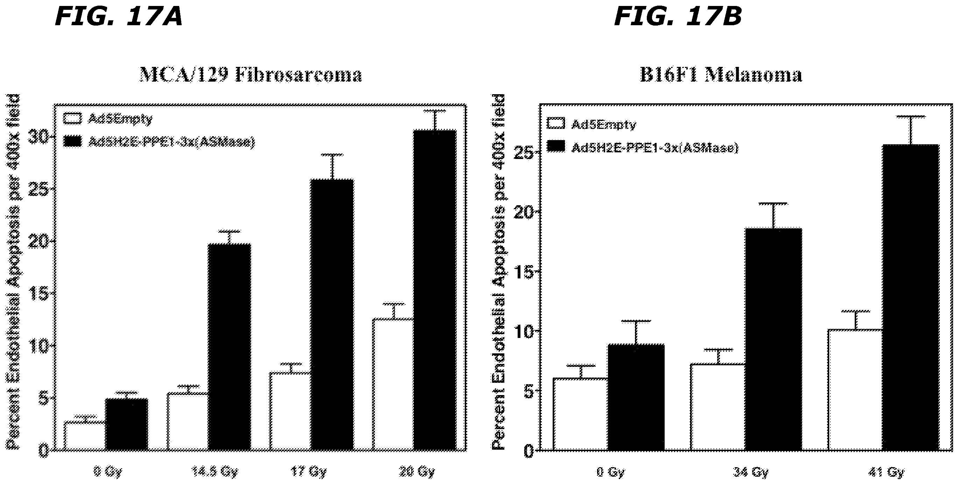

FIG. 17 depicts that overexpression of ASMase in tumor microvasculature leads to an increase in endothelial apoptosis in MCA/129 fibrosarcoma and B16F1 melanoma tumors. 1x10¹⁰ PFU of Ad5Empty or Ad5HEPPE-3x(ASM) was administered intravenously to MCA/129 fibrosarcoma- (FIG. 17A) and B16F1 melanoma- (FIG. 17B) bearing SV129/C57^{*asm*+/+*JAX*} mice. Five (FIG. 17A) or four (FIG. 17B) days post virus administration, tumors were locally irradiated with 14.5, 17 Gy and 20 Gy (FIG. 17A) or 34 and 41 Gy (FIG. 17B), and apoptosis was quantified following TUNEL/Meca-32 immuno-staining. Data (mean ± SE) represent TUNEL-positive endothelial cells quantified from 20 400x magnification fields from an experiment employing 2 animals per group.

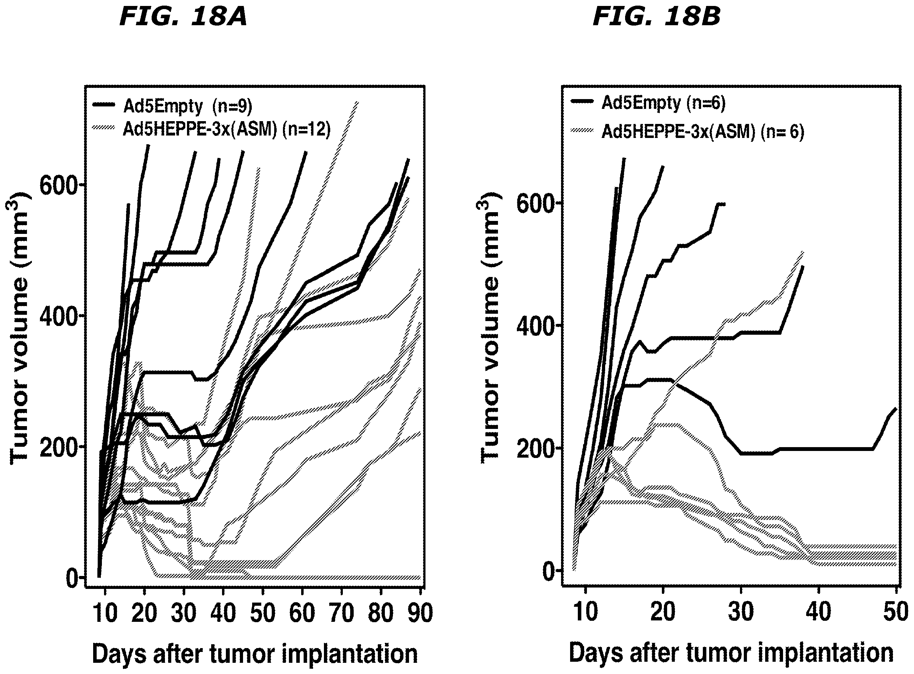

FIG. 18 depicts that overexpression of ASMase in tumor endothelium radiosensitizes B16F1 melanoma. 1x10¹⁰ PFU of Ad5Empty or Ad5HEPPE-3x(ASM) was administered intravenously to B16F1 melanoma-bearing SV129/C57^{*asm*+/+*JAX*} mice. Four days post virus administration, tumors were locally irradiated with 34 (FIG. 18A) and 41 Gy (FIG. 18B). Response of B16F1 melanoma to treatment with Ad5Empty (black lines) or Ad5HEPPE-3x(ASM) (gray lines) and IR is presented as tumor volume. Tumors were measured daily up to 40 days and twice weekly thereafter. Tumor regression was confirmed by local biopsy.

### DETAILED DESCRIPTION OF THE INVENTION

Ceramide is an N-acylsphingosine consisting of a fatty acid bound to the amino group of the sphingoid base, sphingosine. In nature, ceramides are found with fatty acids of various lengths, containing 2 to 28 carbon atoms. Depending on cell type and stimulus, ceramide can be generated either through sphingomyelinase (SMase)-dependent catabolism of sphingomyelin, through a *de novo* synthetic pathway or through a salvage synthetic pathway. SMases are specialized forms of phospholipase C that cleave the phosphodiester bond of sphingomyelin to generate ceramide. Three SMases, distinguishable by their different pH optima, ion dependence and sub-cellular localization, have been identified.

Biologically, ceramide acts as a second messenger in ubiquitous, evolutionarily conserved signaling pathways, including apoptosis, growth arrest, senescence and differentiation. Most attention has been focused on the role of ceramide in stress-induced apoptosis as increased ceramide levels are observed preceding biochemical and morphologic manifestations of apoptosis in a number of cell systems. Addition of exogenous ceramide or sphingomyelinase, as well as pharmacological agents that interfere with enzymes catalyzing the breakdown of ceramide, mimic the effects of stress stimuli and apoptosis. Moreover, cells derived from subjects with Niemann-Pick disease, an inherited deficiency in ASMase activity, show abnormalities in stress-induced apoptosis, supporting the role of ceramide generation, and ASMase, in apoptosis. Finally, the evolutionarily conserved role of ceramide in stress response signaling has been shown in *Saccharomyces cerevisiae.* Upon heating, S. *cerevisiae* mutants incapable of rapid ceramide generation do not adapt and grow at elevated temperatures, as opposed to wild-type counterparts. Exogenous addition of ceramide reverses this phenotype, suggesting that ceramide signaling may constitute a programmed stress response that evolutionarily predates apoptosis.

ASMase is the best characterized SMase, shown to be critically involved in many forms of cellular activation and ceramide-mediated membrane reorganization. While ASMase was originally considered strictly lysosomal because of its pH optimum at 4.5 - 5.0, it is now known that ASMase also localizes to secretory vesicles at the plasma membrane. Because only the on and off rate of the substrate, rather than the catalytic activity of the enzyme, is regulated by pH, ASMase can also hydrolyze sphingomyelin at the neutral pH found on the cell surface, albeit with lower efficiency. Furthermore, the enzyme exists in two forms, termed lysosomal SMase (L-ASMase) and secretory SMase (S-ASMase), differing in their glycosylation pattern and NH2-terminal processing, and hence in their subcellular localization. However, L-ASMase and S-ASMase are derived from the same gene and a common protein precursor of 629 amino acids.

Studies have provided evidence that, in addition to DNA damage, ionizing radiation can act upon cellular membranes to initiate apoptotic death in some cells. Genetic, biochemical and cell biological data have established a critical role for ASMase-mediated ceramide generation in radiation-induced apoptosis, in particular in endothelial cells *in vitro* and *in vivo.* The present inventors have developed an adenoviral delivery system to overexpress human ASMase specifically in endothelium *in vitro* and *in vivo.* Tissue specificity was achieved by using a modified pre-proendothelin-1 promoter, PPE-1(3x), which leads to preferential expression in angiogenic endothelial cells. These constructs increase target gene expression in endothelial cells *in vitro* with minimal expression in cells of non-endothelial origin. The cell culture studies demonstrate that PPE-1(3x)-mediated ASMase overexpression results in enhanced secretory and lysosomal ASMase activity, with a concomitant increase in ceramide generation and CRP formation and that ASMase overexpression leads to an increase in radiation-induced endothelial apoptosis in a time- and a dose-dependent manner, providing proof-of-principle that ASMase radiosensitizes endothelium. ASMase, and presumably CRPs, mediate microvascular apoptosis in responses to irradiation and in turn, tumor response to single-dose radiotherapy. ASMase mediated early-phase microvascular endothelial apoptotic injury is mandatory for tumor regression. Therefore, restoring or amplifying ASMase activity and CRP formation in the endothelium would radiosensitize tumors. Overexpression of ASMase beyond physiological levels in tumor endothelium of wild-type SV129/C57BL/6 resulted in an enhanced tumor response to radiation leading to an increase in tumor regression in a dose-dependent manner. Therefore, modulating ceramide signaling by genetic upregulation of ASMase within tumor vasculature can radiosensitize these tumors, improving tumor response.

Therefore, disclosed herein are ASMase upregulating agents such as the disclosed ASMase upregulating construct.

In an additional embodiment, the ASMase upregulating construct can be targeted to the tumor, such as to the tumor vasculature, by association of the ASMase upregulating construct with a targeting molecule such as a monoclonal antibody specific for a tumor marker.

In one embodiment, administration of the ASMase upregulating agent disclosed herein will reduce the amount of radiation necessary to treat the tumor compared to the amount of radiation necessary in the absence of the construct.

In another embodiment, administration of the ASMase upregulating agent disclosed herein along with at least one radiation dose will cause regression of at least one tumor or decrease in tumor burden.

The ASMase upregulating agent disclosed herein is administered such that it enters the patient's cells and results in ASMase being upregulated in the tumor endothelial cells (tumor endothelium/vasculature). The ASMase upregulating agent may be administered to patients or experimental animals with a pharmaceutically-acceptable diluent, carrier, or excipient, in unit dosage form. Conventional pharmaceutical practice may be employed to provide suitable formulations or compositions to administer such compositions to patients or experimental animals. Although intravenous administration is preferred, any appropriate route of administration may be employed, for example, parenteral, subcutaneous, intramuscular, intracranial, intraorbital, ophthalmic, intraventricular, intracapsular, intraspinal, intracisternal, intraperitoneal, intranasal, aerosol, or topical (e.g., by applying an adhesive patch carrying a formulation capable of crossing the dermis and entering the bloodstream) administration. Therapeutic formulations may be in the form of liquid solutions or suspensions; and for intranasal formulations, in the form of powders, nasal drops, or aerosols. Any of the above formulations may be a sustained-release formulation.

In another embodiment, the ASMase upregulating agent is delivered by a pump. Such pumps are commercially available, for example, from Alzet (Cupertino, Calif.) or Medtronic (Minneapolis, Minn.). The pump may be implantable. Another convenient way to administer the ASMase upregulating agent is to use a cannula or a catheter.

In the disclosed methods, radiation is administered in one or more individual doses in the amount of 0.1-30 Gy, alternatively 0.2-24 Gy or 0.3-16 Gy, but not always limited thereto and can be regulated by an experienced doctor with consideration of age, height and weight of a patient, severity of disease, target area and excretion.

A radiosensitization method described herein may include radiation produced by an X-ray beam or electron beam produced by a linear accelerator. The radiotherapy intended in the present methods may be carried out through a protocol which is generally employed in this technical field and known to those skilled in the art. For example, the radiotherapy includes radiation of cesium, iridium, iodine, or cobalt. The radiotherapy may be systemic radiation (to acute leukemia, malignant lymphoma, and a certain type of solid cancer), but is preferably locally focused on site(s); i.e., tumor sites and solid cancer tissues (abdomen, lung, liver, lymph nodes, head, etc.). The radiotherapy of the present method is administered after radiosensitization (administration of the ASMase upregulating agent), with at least one radiation dose per radiosensitizer dose. In alternative embodiments, the ASMase upregulating agent may be administered multiple times over the course of a patient's therapy.

In another embodiment, the method may further comprise administration of an anti-tumor agent including, but not limited to, platinum-containing drugs, taxane drugs, vinca alkaloid drugs, topoisomerase inhibitors, antimetabolites, and alkylating agents. More specific examples include one or more species of cisplatin, carboplatin, oxaliplatin, paclitaxel, docetaxel, vincristine, vinblastine, vinorelbine, vindesine, irinotecan hydrochloride, topotecan, etoposide, teniposide, doxorubicin, fluorouracil, tegafur, doxifluridine, capecitabine, gemcitabine, cytarabine, methotrexate, pemetrexed, cyclophosphamide, adriamycin, and mitomycin. When said other anti-tumor agents are employed in combination, age, sex, degree of symptom and adverse effects of patients, contraindication upon mixing, etc. are taken into consideration.

### EXAMPLES

### MATERIALS AND METHODS

**Cell Culture and Stimulation.** Human umbilical vein endothelial cells (HUVEC) and human coronary artery endothelial cells (HCAEC), obtained from Cambrex were cultured in EBM-2 medium supplemented with EGM-2 or EGM-2 MV SingleQuot supplement, respectively (Cambrex) at 37°C in a humidified 5% CO₂ chamber. HeLa cells, obtained from the American Type Culture Collection (ATCC), were cultured in DMEM supplemented with 10% fetal bovine serum (FBS), 100 U/ml penicillin, 100 µg/ml streptomycin, and 2 mM L-glutamine at 37°C in a humidified 5% CO₂ chamber. Cloned populations of bovine aortic endothelial cells (BAEC) were cultured in DMEM supplemented with 5% normal calf serum (NCS), 100 U/ml penicillin, 100 µg/ml streptomycin, and 2mM L-glutamine at 37°C in a humidified 10% CO₂ chamber. Upon reaching confluence, cells were cultured in DMEM supplemented with 2% NCS for a minimum of one week. Prior to irradiation experiments, BAEC were pre-incubated for 18 hours in DMEM containing 0.2% human albumin. Irradiation of cultured cells was carried out in Shepherd Mark I irradiator containing a ¹³⁷Cs source at a rate of 2.08 Gy/minute. For experiments involving examination of events occurring under 10 minutes, irradiation was carried out closer to the ¹³⁷Cs source at a rate of 13.1 Gy/minute.

Where indicated, cells were pre-incubated with 1 µg/ml mouse monoclonal anti-ceramide antibody MID15B4 (Alexis Biochemicals) 15 minutes prior to irradiation. In each study, an aliquot of cells were stained with trypan blue to assess viability.

**Apoptosis Quantification.** Apoptosis was assessed *in vitro* by examining morphologic changes in the nuclear chromatin. Stimulated cells were fixed with 2% paraformaldehyde, washed with phosphate buffered saline (PBS), and stained with 100 µl of 24 µg/ml bis-benzimide trihydrochloride solution (Hoechst #33258) for 10 minutes. Morphologic changes of nuclear apoptosis including chromatin condensation, segmentation and compaction along the periphery of the nucleus or the appearance of apoptotic bodies were quantified using an Axiovert S-100 Zeiss fluorescence microscope. A minimum of 400 cells were examined per point.

Apoptosis was quantified *in vivo* in the endothelium of tumor samples following terminal deoxytransferase-mediated deoxyuridine triphosphate nick end labeling (TUNEL) staining. Several different endothelial markers were evaluated for use on 5 µm paraffin embedded sections in combination with TUNEL; the best signal to noise ratio was achieved with a monoclonal antibody against the endothelial cell surface marker MECA-32 (Developmental Studies Hybridoma Bank, The University of Iowa, IA).

**Detection of Ceramide-rich Platforms.** Platforms were detected as previously described (Rotolo, J.A., et al., Caspase-dependent and -independent activation of acid sphingomyelinase signaling. J Biol Chem, 2005. 280:26425-34). Briefly, confluent BAEC were detached by incubation with PBS supplemented with 0.1% collagenase, 0.02% EDTA and 0.5% BSA at 37°C for 5 minutes. Detached cells were gently dispersed mechanically to obtain a single cell suspension and re-suspended at 0.5 x10⁶/ml in DMEM supplemented with 0.2% human albumin. Tumor endothelial cells, following elution from the MACS separation column, were washed with PBS and re-suspended at 0.3 x10⁶/ml in DMEM supplemented with 0.2% human albumin. Following stimulation with irradiation, cells were incubated at 37°C for the time periods indicated and fixed with 2% paraformaldehyde for 15 minutes at 4°C. Prior to staining, non-specific sites were blocked by incubation in PBS containing 2% FBS for 20 minutes. Following a PBS wash, cells were stained for surface ceramide or surface ASMase, using a mouse monoclonal anti-ceramide antibody MID 15B4 IgM (1:50 dilution, Alexis Biochemicals) or polyclonal rabbit anti-ASMase antibody 1598 (1:100 dilution) respectively, for 1 hour at 4°C. Irrelevant mouse IgM or rabbit IgG were used as isotype controls. Following three washes with PBS containing 0.05% Triton X-100, cells were stained for CRP detection with Texas Red-conjugated anti-mouse IgM or Cy3-conjugated anti-rabbit IgG (1:300 dilution, Roche Molecular Biochemicals), respectively, for 1 hour at 4°C. Lastly, cells were washed three times in PBS containing 0.1% Triton X-100 and mounted in fluorescent mounting medium (Dako). Fluorescence was detected using an Axiovert S-100 Zeiss fluorescence microscope equipped with a SPOT digital camera. The percentage of cells containing platforms, i.e. those in which the fluorescence condenses into less than 25% of the cell surface, was determined by counting 150-250 cells per point.

The rabbit polyclonal anti-ASMase antibody 1598 was generated against full-length FLAG-tagged human ASMase protein. Anti-sera was purified over a BIO-RAD T-Gel Column to obtain an IgG fraction that displays specific immunoreactivity by immunoblot assay at a concentration of 100 ng/µl towards 100 ng of purified recombinant human ASMase or ASMase from 25 µg of Jurkat cell lysates. At a concentration of 200 µg/µl, 1598 quantitatively immunoprecipitates ASMase activity from 100 ng of purified ASMase and at a concentration of 200 ng/µl detects cell surface expression of ASMase by flow cytometry or confocal immunofluorescence microscopy.

**Ceramide Quantification.** BAEC, stimulated with ionizing radiation (IR), were incubated for the indicated times at 37°C. Stimulation was terminated by placing the cells on ice. Subsequently, cells were washed twice with cold PBS, and lipids were extracted by addition of scraped cells in methanol to an equal volume of chloroform and 0.6 volume of buffered saline solution/EDTA solution (135 mM NaCl, 4.5 mM KCI, 1.5 mM CaCl₂, 0.5 mM MgCl₂, 5.6 mM glucose, 10 mM HEPES pH 7.2, 10 mM EDTA). Ceramide was quantified using the diacylglycerol (DG) kinase assay.

**ASMase Activity Measurement.** ASMase activity was quantified in BAEC by radioenzymatic assay using [¹⁴C-methylcholine]sphingomyelin (Amersham Biosciences) as substrate, as described with minor modifications (Schissel, S.L., et al., Zn2+-stimulated sphingomyelinase is secreted by many cell types and is a product of the acid sphingomyelinase gene. J Biol Chem, 1996. 271:18431-6). Briefly, following stimulation, the cells were placed on ice at indicated time points. Conditioned media containing proteins secreted over a period of 18 hours was collected, filtered through 40 µm filter mesh (BD Falcon) and concentrated 10-fold using Amicon Ultracel- 30 (Millipore) concentrator (molecular weight cut off, 30,000). Cells were washed with ice cold PBS and subsequently lysed in PBS containing 0.2% Triton X-100. For assaying the activity, post nuclear supernatants or conditioned media were incubated with the substrate in 250 mM sodium acetate, pH 5.0 supplemented with 0.1% Triton X-100 and 1 mM EDTA (cellular homogenates) or 0.1 mM ZnCl (conditioned media). Subsequently, as indicated in figure legends, different combinations of EDTA and ZnCl were used to determine the dependence of the cellular or secreted ASMase activity on extracellular Zn²⁺. Reactions were terminated after 1 hour with CHCl₃:MeOH:1N HCl (100:100:1, v:v:v), and product was quantified with a Beckman Packard 2200 CA Tricarb scintillation counter.

**ASMase Surface Expression.** BAEC were detached from tissue-culture dishes as described for detection of CRPs, and ASMase activity was assayed by flow cytometric analysis. Following detachment and a wash with ice cold PBS, non-specific sites were blocked by a 15 minute incubation with CD16/CD32 FcR block (BD Biosciences). Cells were re-washed, incubated for 45 minutes with 1 µg/ml of isotype control rabbit IgG or polyclonal anti-ASMase 1598 antibody in PBS supplemented with 0.5% FBS, followed by washing and incubation with Cy3-conjugated anti-rabbit IgG in PBS supplemented with 0.5% FBS. 20,000 cells were analyzed on a FACScan flow cytometer (BD Biosciences) with CellQuest software (Becton Dickinson).

**Preparation of Recombinant Replication-deficient Adenoviruses HEPPE-3x(GFP) and HEPPE-3x(ASM).** The murine pre-proendothelin-1 3x (PPE-3x) promoter was ligated into the BamHI/Notl restriction site of the shuttle vector. Subsequently, the HIF2α-Ets-1 enhancer was ligated into the HindIII restriction site of the shuttle vector upstream from the PPE-1(3x) promoter. Human ASM gene (accession number M59916), originating from PCMV1 (ASM) (Genzyme) and GFP gene (accession number U55761), originating from pEGFP-1 (Clontech) were ligated downstream from the HIF2α-Ets-1 enhancer/PPE-1(3x) promoter cassette within the shuttle vector. Lastly, HIF2-Ets-2α/PPE-1(3x)/hASM or HIF2-Ets-2α/PPE-1(3x)/GFP cassette was subcloned into the Mlu-1 restriction site generated within the MCS of pVQAs-NpA vector (Viraquest, Inc). The replication-deficient recombinant adenoviruses (serotype 5) termed Ad5HEPPE-3x(GFP) or Ad5HEPPE-3x(ASM) were prepared using the RAPAd.I system. Viral stocks were stored at - 80°C at concentration of 10⁹-10¹¹ plaque-forming units/ml (PFU/ml).

Adenoviruses used as empty vector control, Ad5Empty, or non-tissue-specific control, Ad5CMV(GFP), were purchased from Viraquest Inc.

**Adenovirus *In Vitro* Infections.** BAEC (100,000 cells/well), HUVEC, HCAEC and HeLa cells (70,000 cells/well) were carefully counted and plated in 12-well tissue culture treated plates 24 hours before infection. Prior to plating, cells were resuspended in their respective culture media, as indicated above, supplemented with 10% NCS (BAEC) or 10% FBS (HUVEC, HCAEC and HeLa). Infections were performed by incubation with 1, 5 and 10 MOI of Ad5Empty or Ad5HEPPE-3x(GFP) in a total volume of 400 µl of culture media supplemented with 2% NCS (BAEC) or 2% FBS (HUVEC, HCAEC and HeLa). After 12 hours, virus-containing media was removed and cells were incubated with culture media supplemented with 5% NCS (BAEC) or 10% FBS (HUVEC, HCAEC and HeLa) in a total volume of 1 ml. At indicated times, cells were detached by a 2 minute incubation in 0.05% trypsin (Cambrex) and resuspended in PBS supplemented with 0.5% FBS, and GFP expression was assessed by flow cytometric analysis. 7-AAD Viability Dye (BD Biosciences) was used to quantify dead cells. 20,000 cells were analyzed on a FACScan flow cytometer with CellQuest software.

**Mice and *In Vivo* Experiments.** SV129/C57BL/6^{*asm*-/-} mice were inbred in the inventors' colony and genotyped using a revised PA2 primer (5'-GGCTACCCGTGATATTGC-3', SEQ ID NO:1), and 35 cycles of PCR amplification, each at 94°C for 15 seconds, 64°C for 30 seconds, and 68°C for 90 seconds. Wild-type, SV129/C57BL/6^{asm+/+JAX} male mice, 6-8 weeks old, were purchased from Jackson Laboratories. Mice were housed at the animal core facility of Memorial Sloan-Kettering Cancer Center. This facility is approved by the American Association for Accreditation of Laboratory Animal Care and is maintained in accordance with the regulations and standards of the United States Department of Agriculture and the Department of Health and Human Services, National Institutes of Health.

Experiments with asmase+/+ mice utilized the commercially-available SV129/BL6 mouse strain from Jackson Laboratories, which is termed SV129/BL6^{JAX}, as host. This strain displays significantly greater resistance to endothelial cell apoptosis than the inventors' in-house bred SV129/BL6^{SKI} strain for unknown reasons and right-shifts tumor responses (not shown). Hence the 50% tumor control dose (TCD50) for fibrosarcomas increases from -15 Gy in SV129/BL6^{SKI} hosts to >30 Gy in SV129/BL6^{JAX} hosts, while a complete regression of melanoma is not induced in either background. However, the SV129/BL6^{JAX} strain has the virtue of strict batch-to-batch stability as these commercial mice are the product of heterozygous mating of pure SV129 and C57BL6 mouse strains, while the inventors' in-house propagated colony is interbred as an SV129/BL6 strain and is thus subject to genetic drift.

MCA/129 fibrosarcoma and B16F1 melanoma cells were maintained in DMEM high glucose supplemented with 10% FBS, 100 U of penicillin/ml and 100 mg of streptomycin/ml in 10% CO₂ at 37°C. The cells (10⁶) were resuspended in PBS and injected subcutaneously into the right flank. For adenovirus administration optimization experiments, once tumors reached an indicated size, 5x10⁹ or 1x10¹⁰ PFU of Ad5Empty, Ad5HEPPE-3x(GFP) or Ad5CMV(GFP) was administered by various methods. For intravenous administration, 200 µl of adenovirus was delivered by a single tail vein injection. For intratumoral administration, mice were lightly sedated with ketamine (0.1 mg/g) and xylazine (0.02 mg/g), and adenovirus was injected intratumorally using a Hamilton microsyringe with a 26-gauge needle. Four injections, 10 µl of viruses per track, were used to improve the distribution of the viruses within the tumors. For administration by osmotic pump, mice were lightly sedated, an Alzet osmotic pump containing 200 µl of appropriate adenovirus was surgically placed adjacent to the tumor and tumors were continuously infused with the adenovirus over a period of 24 hours.

In the radiation experiments, 1x10¹⁰ PFU of Ad5Empty or Ad5HEPPE-3x(ASM) was delivered intravenously to mice bearing tumors 80-100 mm³ in size. Five days post virus administration, an indicated dose of radiation was delivered using a Philips MG-324 X-ray unit at 105.5 cGy/minute (50 cm source to skin distance). Mice were lightly sedated with ketamine (0.1 mg/g) and xylazine (0.02 mg/g). and only tumor, surrounding skin and subcutaneous tissues were exposed; the rest of the mouse was shielded using a specialized lead jig. Tumor volume, based on caliper measurements, was calculated daily.

**Endothelial Cell Isolation.** Tumor endothelial cells were isolated following a modification of a technique published by Garcia-Barros *et al.* (Garcia-Barros, M., et al., Tumor response to radiotherapy regulated by endothelial cell apoptosis. Science, 2003. 300:1155-9). MCA 129/fibrosarcoma tumors were dissected from the hind limbs, washed twice in PBS, cut into small pieces and incubated in a cocktail containing 2 mg/ml collagenase A (Roche), 250 µg/ml elastase (Roche) and 25 µg/ml DNAsel (Roche) in DMEM supplemented with 1% FCS, 20 mM HEPES (pH 7.4),100 U/ml penicillin and 100 µg/ml streptomycin at 37°C with gentle shaking. After 45 minutes, the tumor digest was filtered sequentially through 100, 70 and 40 µm nylon filter mesh (BD Falcon). Filtered samples were washed twice with 0.5% BSA in PBS and centrifuged at 800xg for 5 minutes three times at 4°C. Cells were separated based on density through a preformed 30% Percoll gradient (Amersham Pharmacia Biotech) at 800xg for 30 minutes at 4°C. This step removes platelets and red blood cells, which can cause clumping of the magnetic beads. Cells at the top of the gradient were removed carefully, and washed twice with 0.5% BSA in PBS. For negative selection, to remove hematopoietic cells, MACS microbeads (Miltenyi Biotec Inc.), conjugated to antibody directed against hematopoietic cell surface marker CD45 were incubated with the fraction obtained from the Percoll gradient for 15 minutes at 4°C at a 1:10 dilution, as indicated by the manufacturer. The total Percoll gradient fraction-antibody-conjugated MACS microbeads incubation was applied to the MACS LS Separation columns (Miltenyi Biotec Inc.), and the column was washed with 9 ml of 0.5% BSA in PBS. This process was repeated using the flowthrough to increase specific binding. Flow cytometric analysis showed that 90% of the cells retained on the column were positive for CD45. Thereafter, the effluent fraction was incubated with MACS microbeads conjugated to anti-mouse CD146 antibody (LSEC microbeads; Miltenyi Biotech) for positive selection of tumor endothelial cells (1:10 dilution). Following a 15 minute incubation at 4°C, cells were washed and applied to the MACS LS Separation columns, as described above. Tumor cells pass through the column, while endothelial cells remain bound to the beads. Thereafter, the column was detached from the magnet, and the endothelial cells bound to microbeads were eluted in 0.5% BSA in PBS and subsequently passed through the column to increase specific binding. Analysis by flow cytometry showed that the final eluate from the magnetic column contained 80-90% pure endothelial cells based on the binding of endothelial specific markers VEGFR2, CD31 (BD Biosciences) and VE-cadherin (clone Bv13, ImClone).

**Tissue GFP Expression Quantification.** To test the cellular distribution of the delivered GFP *in vivo,* tissues were dissected at indicated times after *in vivo* infection, washed with PBS and fixed in freshly prepared 4% paraformaldehyde in PBS at 4°C overnight. Following paraffin embedding, 5 µm thick sections were obtained by microtomy, adhered to polylysine-treated slides and deparaffinized by heating at 90°C for 10 minutes and at 60°C for 5 minutes, followed by two xylene washes for 5 minutes. Automated immunostaining (Discovery XT automated machines) of the tissue sections was performed using 10 µg/ml of a rabbit polyclonal anti-GFP antibody (Molecular Probes). To quantify GFP expression in endothelium, GFP-stained tissues were subsequently stained with 3 µg/ml of monoclonal antibody against the endothelial cell surface marker MECA-32 (Developmental Studies Hybridoma Bank, The University of lowa). Fluorescence was detected using an Axiovert S-100 Zeiss fluorescence microscope equipped with a SPOT digital camera. For quantifying GFP-positive endothelial cells, immunostained slides were scanned using the Mirax scanner and generated images were analyzed using the Mirax viewer software (Carl Zeiss, Inc.)

**Crypt Microcolony Survival Assay.** The microcolony survival assay was performed as previously described (Rotolo J.A., 2005). Briefly, small intestinal samples were obtained 3.5 days after irradiation, and 2.5-cm segments of proximal jejunum were obtained at 2 cm from the ligament of Trietz and fixed in freshly prepared 4% paraformaldehyde in PBS at 4°C overnight. Following paraffin embedding, transverse tissue sections of the full jejunal circumference (5 µm thick) were obtained by microtomy from the paraffin blocks, adherence to polylysine-treated slides, and deparaffinizing by heating at 90°C for 10 minutes and at 60°C for 5 minutes, followed by two xylene washes for 5 minutes, and staining with hematoxylin and eosin according to a standard protocol. Crypts were identified histologically according to the criteria established by Withers and Elkind (Microcolony survival assay for cells of mouse intestinal mucosa exposed to radiation. Int J Radiat Biol Relat Stud Phys Chem Med, 1970. 17:261-7). Surviving crypts were defined as containing 10 or more adjacent chromophilic non-Paneth cells, at least one Paneth cell, and a lumen. The circumference of a transverse cross-section of the intestines was used as a unit. The number of surviving crypts was counted in each circumference. Ten to twenty circumferences were scored per mouse, and 2-4 mice were used to generate each data point.

**Statistics.** Values are expressed as mean ± standard deviation unless otherwise noted. Paired, two-tailed Student's t tests were calculated using Prism v4. P values less than 0.05 were considered to be significant.

### Example 1

### Endothelial-specific adenoviral vectors containing a reporter gene, GFP, or a therapeutic gene, ASMase

In order to efficiently deliver targeted genes to endothelium, two adenoviral agents were developed to express GFP [Ad5HEPPE-3x(GFP)] and ASMase [Ad5HEPPE-3x(ASM)] in both cell culture and *in vivo* models. The Ad5HEPPE-3x(ASM) construct is also referred to as Ad5H2E-PPE1-3x(ASMase) and as Ad5H2E-PPE1 (3x)-ASMase. Ad5HEPPE-3x(ASM), Ad5H2E-PPE1-3x(ASMase) and Ad5H2E-PPE1(3x)-ASMase all refer to the same construct. Adenovirus was chosen as a vehicle because of its affinity to Coxsackie adenovirus receptors (CAR), receptors ubiquitously expressed on almost all cell types, and because it is internalized via αᵥβ₅ and αᵥβ₅ integrins, which display high expression in angiogenic endothelial cells. Further, adenoviruses are stable, have high infection efficiency and are relatively easily manipulated and produced at a high titer. Ad5HEPPE-3x(GFP) was utilized as a reporter system to confirm specificity of the adenoviral agent to endothelial cells and optimize its delivery *in vitro* and subsequently *in vivo.* ASMase was inserted into the virus construct as a therapeutic gene, generating Ad5HEPPE-3x(ASM), in order to study whether overexpression of ASMase would result in an increase in the sensitivity of tumor endothelium to single-dose radiotherapy.

The schematic representation of the endothelial-specific adenoviral vectors is depicted in FIG. 1. As shown in the schematic, a cassette containing a hypoxia-inducible enhancer (HIF-2α-Ets-1), an endothelial-specific promoter [a modified murine pre-proendothelin-1 (PPE-1) promoter, PPE-1(3x)] and either a reporter gene (GFP, FIG. 1A) or a therapeutic gene (human ASMase, FIG. 1B) was inserted into a replication-deficient adenovirus serotype 5. The vectors were designed to target gene expression specifically to the endothelium and more specifically to enhance expression under the hypoxic conditions characteristic of tumors. PPE-1(3x), a 1.5 kb promoter, was originally generated from the wild-type PPE-1 promoter. Target gene expression controlled by the PPE-1 promoter was 15-30 times higher in endothelial cells *in vitro* than target gene expression controlled by the constitutive cytomegalovirus (CMV) promoter. Furthermore, PPE-1 promoter-controlled gene expression was 60 times higher in endothelial cell lines than in non-endothelial cell lines, confirming its preferential activity in the endothelium. Modification of the promoter to incorporate three copies of the endothelial cell positive regulatory cis element ETC/D/E [PPE-1 (3x)] led to an increase in the specificity and efficiency of promoter-driven target gene expression. Specifically, when compared to PPE-1, PPE-1 (3x) led to an additional 2.5-25-fold increase in target gene expression in endothelial cell lines *in vitro* and a 3.5-4-fold increase in expression in tumor endothelium *in vivo.* Additionally, only minimal activity of the PPE-1 (3x) promoter was observed in the endothelium of normal tissues, making it an ideal candidate to target expression of the therapeutic gene of interest, ASMase, specifically to tumor endothelium.

The PPE-1 promoter contains a hypoxia-responsive element, starting at 118 base pairs upstream of the transcription start site that increases expression from the promoter under hypoxic conditions. In order to further boost target gene expression specifically in hypoxic environments such as those characteristic of tumors, a 132 base pair dual binding element, HIF-2α-Ets-1, was inserted upstream from the promoter. This enhancer was originally found in the VEGFR-2 promoter region and enhances VEGFR-2 transcription during vasculogenesis.

### Example 2

### Characterization of specificity, efficacy and time course of Ad5HEPPE-3x(GFP) infection

In order to characterize the generated adenoviruses, initial studies focused on the Ad5HEPPE-3x(GFP) virus which utilized GFP as a reporter gene. BAEC were infected with a range of doses of the adenovirus (MOI = 1-10), and flow cytometric analysis was used to determine the maximal infection efficiency. Maximal efficiency was achieved following infection at MOI=5, which corresponds to five viral plaque forming units (PFU) per cell. At this concentration, 90.2±4.8% of BAEC expressed GFP 72 hours post infection (data not shown), demonstrating efficient viral transduction and target gene expression. In order to test the efficacy and specificity of Ad5HEPPE-3x(GFP), a number of endothelial and non-endothelial cell lines were infected with increasing doses of the virus. While infection with Ad5HEPPE-3x(GFP) lead to GFP expression in 90.6%, 77.6% and 88.9% of BAEC, HUVEC and HCAECs, respectively, it only induced GFP expression in 2.6% of HeLa cells (FIG. 2) and 1.6% of Jurkat cells (data not shown). These data confirm that infection with the Ad5HEPPE-3x construct results in high efficiency, high specificity reporter gene expression in endothelial cells *in vitro.*

Subsequently, the time course of infection was analyzed in BAEC infected with MOI=5 of Ad5HEPPE-3x(GFP) by flow cytometry. As shown in FIG. 3A, maximal GFP expression was achieved at 72 hours post-infection and was maintained for at least four additional days. Moreover, no significant adenovirus-induced toxicity was observed in infected cells up to 7 days post-infection (FIG. 3B), as analyzed by 7AAD viability dye inclusion. These data were important in setting the parameters for the subsequent set of studies in which a therapeutic protein, ASMase, was expressed by an analogous delivery system, referred to as Ad5HEPPE-3x(ASM).

### Example 3

### Overexpression of ASMase via Ad5HEPPE-3x(ASM) leads to an increase in ASMase activity, ceramide generation and CRP formation in endothelial cells

Upon optimization of virus infection of BAEC by Ad5HEPPE-3x(GFP), the effects of Ad5HEPPE-3x(ASM) were examined. In order to determine whether adenovirus-mediated overexpression of ASMase leads to generation of a physiologically active enzyme, ASMase activity was assessed in BAEC infected with Ad5HEPPE-3x(ASM). The ASMase gene gives rise to two forms of the enzyme, lysosomal ASMase (L-ASMase) and secretory ASMase (S-ASMase). These enzyme isoforms differ in their glycosylation pattern and NH₂-terminal processing, resulting in different subcellular targeting. Endothelial cells are a particularly rich source of S-ASMase, secreting 20 times more active enzyme than any other cell type thus far investigated. For this reason, ASMase activity in both cellular homogenates and conditioned media was assayed after cells were infected with Ad5HEPPE-3x(ASM) and compared to the baseline levels found in cells infected with Ad5Empty. As shown in FIG. 4A, expression of ASMase under the control of a PPE-1(3x) promoter lead to an 8.3-fold increase in enzyme activity in the cellular homogenates over baseline (increase from 5.7±1.1 to 47.3±5.0 nmol/hour; p<0.005) and a 46.1-fold increase in S-ASMase activity from baseline in the conditioned media from cells infected with the Ad5HEPPE-3x(ASM) (increase from 4.9±0.3 to 226.4±23.3 nmol/hour; p<0.005). Therefore, similarly to Ad5HEPPE-3x(GFP), Ad5HEPPE-3x(ASM) efficiently infects BAEC. Further, these studies demonstrate that Ad5HEPPE-3x(ASM) delivers the human ASMase gene and that the gene is properly expressed and processed by the cellular machinery to generate enzymatically active protein.

Both lysosomal and secretory forms of ASMase are metalloenzymes containing several highly conserved Zn²⁺ binding motifs and requiring Zn²⁺ for their activity. However, while L-ASMase is exposed to Zn²⁺ during trafficking to lysosomes or in lysosomes (and/or during cellular homogenization) and is tightly bound to this co-factor, S-ASMase requires the addition of exogenous Zn²⁺ for *in vitro* activity. In order to confirm the Zn²⁺ dependence of the secretary form that rises from adenoviral delivery of the human ASMase, activity of ASMase was assayed in homogenates and conditioned media in the presence and absence of Zn²⁺. As it was shown previously that only a potent chelator, such as 1,10 phenanthroline can strip Zn²⁺ off of the lysosomal enzyme, EDTA was used in instances when extracellular Zn²⁺ was not added in order to ensure that the only metal present in the reaction is that already bound to the enzyme. As shown in FIG. 4B, activity of the secreted endogenous and overexpressed enzyme is dependent on the addition of extracellular Zn²⁺. While the total baseline secreted activity in the media was 4.1±0.4 nmol/hour in the presence of extracellular Zn²⁺, the total activity in the absence of Zn²⁺ was undetectable. Moreover, a 48.5-fold increase in the total activity following ASMase expression was almost completely Zn²⁺-dependent, as only a small fraction of that activity was detected in the absence of the co-factor (189.7±4.5 versus 9.7±4.6 nmol/hour in the presence and absence of Zn²⁺, respectively; p<0.005). On the contrary, the simultaneous control study assaying the total activity in the cellular homogenates showed comparable ASMase activity in the presence and absence of extracellular Zn²⁺, both at the baseline (7.9±0.4 and 7.7±0.3 nmol/hour, respectively; p>0.1) and following infection with Ad5HEPPE-3x(ASM) (31.0±1.8 and 37.0±4.5 nmol/hour respectively; p>0.1). These data indicate that, as is the case for endogenous ASMase, additional ASMase expression driven by the Ad5HEPPE-3x(ASM) construct, does not arise via exocytosis of lysosomes or vesicles in transit to lysosomes but rather via a typical secretory pathway.

Upon determination that overexpression of ASMase by infection with Ad5HEPPE-3x(ASM) leads to significant increases of the lysosomal and secreted ASMase activity *in vitro,* it was determined whether these increased enzyme levels might impact ceramide generation and signal transduction. Initially, BAEC total cellular ceramide content was measured following overexpression of ASMase. As shown in FIG. 5A, overexpression of ASMase via Ad5HEPPE-3x(ASM) led to a 36% (p<0.05) increase in ceramide content in unirradiated cells compared to the Ad5Empty control, as determined by DG kinase assay. Next, the impact of radiation on ceramide generation in Ad5HEPPE-3x(ASM) infected cells was examined. Following exposure to 10 Gy, radiation-induced ceramide elevation in BAEC infected with Ad5HEPPE-3x(ASM) was detected within 1 minute of stimulation and persisted for over 2 minutes before decreasing towards baseline. As shown in FIG. 4A, cells infected with Ad5HEPPE-3x(ASM), and therefore overexpressing ASMase, generated 28.5%, 20.7% and 22.6% more ceramide than cells infected with Ad5Empty at 1, 2 and 5 minutes post radiation, respectively (p<0.05). Infection with Ad5Empty was used as a negative control, and had no effect on cellular ceramide levels when compared to that in uninfected cells (FIG. 5A; p>0.1).

Finally, it was determined whether ASMase overexpression-induced increases in cellular ceramide resulted in a concomitant increase in formation of CRPs, as determined by standard fluorescent microscopy. As in the previous study, Ad5Empty-infected BAEC were utilized as a control and it was determined that infection with an adenovirus per se does not have an effect on CRP formation (FIG. 5B; p>0.1). Overexpression of ASMase, however, led to an increase in the population of cells forming CRPs, both prior to and following exposure to irradiation. Specifically, at baseline, CRPs were detected in 16.4±1.8% of the total population of control cells; however overexpression of ASMase increased the baseline incidence of CRPs to 30.1±2.3% of the total population (p<0.05). Following irradiation, a time-dependent increase in CRP formation was observed. A consistently higher percentage of the total population of Ad5HEPPE-3x(ASM)-infected cells formed CRPs compared to cells infected with Ad5Empty (48.6%±2.7 vs. 31.3%±2.4 at 1 minute; 48.0%±2.4 vs. 35.6%±2.4 at 2 minutes; 40.4%±2.4 vs. 24.4%±2.1 at 5 minutes respectively; p<0.05).

These data collectively show that the delivery of human ASMase gene by the adenoviral vector Ad5HEPPE-3x(ASM) results in a significant increase in lysosomal and secreted ASMase activity in BAEC. Further, the increase in ASMase enzyme activity is translated into a concomitant increase in total cellular ceramide generation and CRP formation, both at baseline and following exposure to radiation.

### Example 4

### ASMase overexpression radiosensitizes endothelial cells

In order to determine the physiological significance of Ad5HEPPE-3x(ASM)-induced increases in ASMase activity and concomitant ceramide generation and CRP formation, the apoptotic response of BAEC was assessed following infection and irradiation. As shown in FIG. 6A, cells exposed to 10 Gy 3 days post infection with Ad5HEPPE-3x(ASM) lead to radiosensitization of BAEC, documented as a 37.8% increase in apoptosis at 8 hours (from 28.0±2.7% to 38.6±3.0% of the total cell population in Ad5Empty- and Ad5HEPPE-3x(ASM)-infected BAEC, respectively; p<0.05).

As determined in adenovirus characterization studies in which Ad5HEPPE-3x(GFP) was utilized, maximal gene expression was achieved three to seven days after infection of the cells. Radiation-induced apoptosis of BAEC was determined daily 8 hours post 10 Gy up to 7 days after infection with adenovirus. These studies showed that an increase in radiation-induced apoptosis of BAEC (53.4%, 30%, 39.4% and 36.2% at 4, 5, 6 and 7 days after infection, respectively; p<0.05 each vs. control) was achieved for the duration of the ASMase maximal expression (FIG. 6A).

Further, the apoptotic response of BAEC infected with Ad5HEPPE-3x(ASM) was studied as a function of time after irradiation with 10 Gy. It was determined that maximal radiosensitization effect was achieved 8 hours after radiation when apoptosis was increased by 33.3% (from 31.5.0±2.0% to 42.1±1.5% of the total cell population in Ad5Empty and Ad5HEPPE-3x(ASM) infected BAEC, respectively; p<0.05). Lastly, the dose dependence of radiosensitization was assessed. As shown in FIG. 6C, overexpression of ASMase induced radiosensitization at doses from 5 to 15 Gy (p<0.05), yielding a dose-modifying factor of 1.35. Overall, these studies showed that overexpression of ASMase via an adenoviral agent confers radiosensitivity to BAEC in a time- and dose-dependent manner.

### Example 5

### Infection with Ad5HEPPE-3x(GFP) induces GFP expression in angiogenic endothelium

In order to test, *in vivo,* the feasibility of administration, efficiency of infection and the specificity for angiogenic endothelial cells of the adenoviruses generated, Ad5HEPPE-3x(GFP) was administered to B16F1 melanoma-bearing C57BL/6 mice and MCA/129 fibrosarcoma-bearing SV129/C57BL/6 mice. The B16F1 melanoma-bearing C57BL/6 mouse model was previously utilized in studies with adenoviral vectors to administer genes expressed under the control of PPE-1 (3x), hence this model was initially used for the optimization studies. Initially, several different routes of viral administration were tested to determine which lead to the highest efficacy and specificity of expression of the reporter gene. To assess the optimal route of administration, 1X10¹⁰ PFU (concentration was determined empirically, data not shown) of Ad5HEPPE-3x(GFP) was delivered to tumor-bearing mice once flank tumors reached approximately 180 mm³ in size. Virus was administered intravenously, intratumorally or via osmotic pump. Five days post-infection, tumors were excised, fixed in paraformaldehyde and embedded in paraffin blocks. Subsequent immunostaining of tumor cross-sections with anti-MECA-32, an endothelial specific antibody that binds a pan-endothelial cell antigen, MECA-32, and anti-GFP antibody was performed, and GFP-positive endothelial cells were counted in twenty 400x magnification fields. These studies determined similar levels of infection efficiency for intravenous and intratumoral administration (5.24%±0.9 and 4.6%±0.7 of the total endothelial population within the tumor, respectively; FIG. 14A; p>0.05). However, intravenous infection did not lead to any detectable expression of GFP in non-endothelial tumor cells while intratumoral infection resulted in GFP expression in tumor cells along the needle track, as well as needle delivery-induced tissue hemorrhage (data not shown). Further, while intravenous administration lead to GFP expression spread evenly throughout the tumor, intratumoral injections failed to spread the virus more than a few millimeters from the injection sites, resulting in detection of GFP expression only along the four needle tracks employed (data not shown). Combination of intravenous and intratumoral injections did not lead to a significant increase in GFP expression over either route individually (5.0%±0.3, p>0.1; FIG. 14A). Finally, osmotic pump-mediated virus administration lead to no detectable GFP expression as determined by examining tumor cross sections immunostained with anti-MECA32 and anti-GFP antibodies five days post viral administration sections (data not shown). These results demonstrate that intravenous route administration of Ad5HEPPE-3x(GFP) leads to the highest infection efficiency and specificity, as well as the best virus distribution throughout the tumor, and was hence used in all subsequent studies.

In order to determine whether tumor size at the time of *in vivo* infection plays a role in infection efficiency, Ad5HEPPE-3x(GFP) was injected intravenously into C57BL/6 mice bearing B16F1 melanoma tumors ranging from 64-203 mm³. Quantification of GFP-positive endothelial cells five days post viral administration, as in the previous study, revealed that tumor size does not play a role in infection efficiency. GFP-positive endothelial cells were observed to be in the range of 4.0%±0.6 to 5.8%±1.1 of the total tumor endothelium (FIG. 7B; p>0.05), irrespective of tumor size. Overall, no correlation was found between the tumor size and infection efficiency.

B16F1 melanoma, as well as MCA/129 fibrosarcoma, have been extensively studied. The vascular component in both tumor models mediates the tumor response to single high dose radiation. However, MCA/129 fibrosarcoma is more sensitive to radiation, demonstrating a 50% regression rate following 15 Gy radiation exposure when implanted in SV129/C57BL/6^{asm+/+} mice. B16F1 melanoma grown in the same background, does not exhibit complete regression following local radiation exposure. Besides the response to radiation, the two tumor models differ in their growth patterns and appearance. While B16F1 melanoma is a fast growing tumor developing necrosis and skin ulcerations at relatively small sizes, MCA/129 fibrosarcoma grows at more predictable rates and is well perfused until reaching sizes above approximately 300 mm³.

In order to test infection efficiency and specificity in the MCA/129 fibrosarcoma tumors, 1X10¹⁰ PFU of Ad5HEPPE-3x(GFP) was delivered intravenously to tumor-bearing SV129/C57BL/6 mice (C57BL/6 background mice do not support growth of MCA/129 fibrosarcoma). As shown in FIG. 7C, comparable levels of GFP expression was observed in MCA/129 fibrosarcoma and B16F1 melanoma tumor models (5.4%±0.9 and 5.9%±0.5 respectively; p>0.05).

To determine the time course of target gene expression in MCA/129 fibrosarcoma-bearing SV129/C57BL/6 mice, GFP expression was examined in tumor sections for 14 days following intravenous administration of 1X10¹⁰ PFU of Ad5HEPPE-3x(GFP). As shown previously, target gene expression under control of PPE-1(3x) peaks in the vasculature 5 days post intravenous adenoviral administration and persists for 14 days. While GFP-positive endothelial cells were detected as early as 2 days post virus administration, peak reporter gene expression was detected 5 days post administration in 4.8%±0.5 of tumor endothelium (FIG. 7D), remaining at a similar level for an additional 9 days (data not shown).

Finally, the specificity of the Ad5HEPPE-3x virus was assessed by determining whether Ad5HEPPE-3x(GFP) infection *in vivo* restricts target gene expression to the angiogenic endothelial bed. As previously shown, PPE-1 (3x) promoter specifically induced expression in the tumor angiogenic vascular bed with a 35-fold higher expression compared to the normal vascular bed of the lung. In the present studies, MCA/129 fibrosarcoma bearing mice were infected with Ad5Empty, Ad5CMV(GFP) or Ad5HEPPE-3x(GFP) virus, and various tissues were harvested for immunofluorescence detection of GFP expression 5 days post viral administration.

Ad5CMV(GFP) was utilized as a tissue non-specific control, as well as a positive control for GFP expression in the liver because hepatocytes exhibit high expression levels of the CAR receptor and hence high affinity for the adenovirus constructs utilized. These characteristics also limit the clinical utility of promiscuous adenoviral-vectors such as Ad5CMV and illustrate the requirement for the generation of tissue-specific expression vectors such as those disclosed herein. As shown in the upper panel of FIG. 8A, administration of Ad5CMV(GFP) leads to high GFP expression in hepatocytes. In contrast, no detectable GFP expression was observed following administration of Ad5Empty or Ad5HEPPE-3x(GFP). Similarly, no detectable GFP expression in endothelium of the GI, heart, kidney, lung, brain (FIG. 8A), spleen, skin or pancreas (data not shown) was observed following intravenous administration of Ad5HEPPE-3x(GFP). While tissues such as the kidney and GI exhibit high levels of autofluorescence, there was no observable increase in green fluorescence expression in these organs in animals infected with any of the three viruses. Alternately, GFP expression similar to levels observed previously, was observed in endothelium of tumors from mice infected with Ad5HEPPE-3x(GFP) (FIG. 8B). However, no detectable GFP expression was observed in tumors of mice infected with Ad5Empty or Ad5CMV(GFP) (FIG. 8B and data not shown). These data corroborate previous results obtained using adenoviral vector-based gene delivery strategies under the control of the PPE-1(3x) promoter. In summary, these studies demonstrate that the Ad5HEPPE-3x adenovirus constructs effectively deliver genes of interest to endothelium *in vivo,* resulting in specific localized gene expression due to the high specificity of PPE-1 (3x) promoter.

### Example 6

### Overexpression of ASMase in endothelium of MCA/129 fibrosarcoma and B16F1 melanoma increased radiation-induced tumor microvascular apoptosis

As depicted in FIG. 17, overexpression of ASMase in tumor microvasculature leads to an increase in endothelial apoptosis in MCA/129 fibrosarcoma and B16F1 melanoma tumors. 1x10¹⁰ PFU of Ad5Empty or Ad5HEPPE-3x(ASM) was administered intravenously to MCA/129 fibrosarcoma- (FIG. 17A) and B16F1 melanoma- (FIG. 17B) bearing SV129/C57^{*asm*+/+*JAX*} mice. Five (FIG. 17A) or four (FIG. 17B) days post virus administration, tumors were locally irradiated with 14.5, 17 Gy and 20 Gy (FIG. 17A) or 34 and 41 Gy (FIG. 17B) and apoptosis was quantified following TUNEL/Meca-32 immunostaining.

Collectively, these data show that genetic upregulation of ASMase not only sensitizes endothelial cells *in vitro* (FIG. 6), but also *in vivo* (FIG 17). Specifically, an increase in ASMase expression in microvasculature of two tumor models sensitizes tumor endothelium to radiation-induced apoptosis.

### Example 7

### Expression of ASMase in tumor endothelium of asmase^{-/-} mice restores sensitivity of MCA/129 fibrosarcomas to radiation

Upon completion of virus characterization using the Ad5HEPPE-3x(GFP) construct, including optimization of dosing and timing of infection and confirmation of gene expression specifically in angiogenic endothelium, the impact of Ad5HEPPE-3x(ASM) on tumor endothelium was examined. Ad5HEPPE-3x(ASM), like the Ad5HEPPE-3x(GFP) construct, was expected to induce expression specifically within tumor endothelium, delivering expression of ASMase to the angiogenic compartment. Because ASMase activity is required to engage the vascular component of the tumor response to radiation, MCA/129 fibrosarcoma implanted into asmase^{-/-} mice (Sloan-Kettering colony) were relatively resistant to radiation doses up to 18 Gy. Further, MCA/129 fibrosarcoma tumors in asmase^{-/-} mice grew 200-400% faster than their wild-type counterparts. Whether restoration of ASMase expression in asmase^{-/-} tumor vasculature would restore the growth pattern and sensitivity to radiation previously observed in wild-type littermates was then studied. Initial experiments assessed the impact of restoration of endothelial ASMase on the radiation response of MCA/129 fibrosarcoma implanted into asmase^{-/-} mice. As shown in FIGs. 9A and 9B, intravenous administration of Ad5HEPPE-3x(ASM) and restoration of ASMase expression in tumor endothelium of asmase^{-/-} mice lead to a 10.5±2.9 day tumor growth delay in comparison to asmase^{-/-} littermates infected with an Ad5Empty construct (p<0.05). Additionally, restoration of ASMase expression in asmase^{-/-} mice significantly sensitized tumor response to radiation. For these studies, asmase^{-/-} mice implanted with MCA/129 fibrosarcoma and infected with Ad5HEPPE-3x(ASM) or Ad5Empty were administered local tumor irradiation 5 days after viral infection (FIG. 9C). Single-dose 15 Gy radiation of MCA/129 fibrosarcoma following Ad5Empty infection induced complete tumor regression in only 1 out of 15 mice (6.6%), and caused a mean tumor growth delay of 15.6±2.9 days in the remaining 14 mice (p<0.05 vs. unirradiated controls; FIG. 9). However, localized tumor exposure to 15 Gy in conjunction with the restoration of ASMase expression via Ad5HEPPE-3x(ASM) infection, resulted in a complete tumor regression in 10 out of 15 mice (66%) and a tumor growth delay in the remaining 5 mice with a mean of 22±6.6 days (p<0.05 vs. unirradiated controls; FIG. 9). These data demonstrate that selective expression of ASMase in the tumor vasculature of asmase^{-/-} mice is able to restore tumor response to radiation to the levels previously observed in wild type littermates (50% local complete tumor regression achieved with a single dose of 15 Gy).

To confirm that Ad5HEPPE-3x(ASM)-induced restoration of radiation sensitivity is mediated by tumor vasculature, endothelial apoptosis of tumor vasculature within MCA/129 fibrosarcoma tumors implanted into asmase^{-/-} mice was assessed 4, 6, 8 and 10 hours post radiation. Briefly, flank tumors were exposed to 15 Gy single-dose radiation and tumors were excised at 6 hours (FIG. 10A) or at the time points indicated (FIG. 10B) after radiation. Following fixation in paraformaldehyde and embedding in paraffin blocks, 5 µm tumor cross-sections were co-stained with an antibody to the endothelial-selective cell surface marker MECA-32 (blue in FIG. 10A) and by terminal deoxytransferase-mediated deoxyuridine triphosphate nick end labeling (TUNEL) for apoptosis (brown in FIG. 10A). Additionally, cross-sections were labeled with hematoxylin to visualize tumor cell nuclei. Quantification of TUNEL-positive endothelial cells revealed that restoration of ASMase expression lead to a time dependent increase in endothelial apoptosis from 3.8±0.6% to 27±2% at 8 hours post 15 Gy (FIG. 10B; p<0.05). On the contrary, local radiation of MCA/129 fibrosarcoma tumors implanted in asmase^{-/-} mice infected with Ad5Empty virus did not result in a significant increase in endothelial apoptosis within the same time frame (5.3±1% vs. 1.2±0.4% in unirradiated control; FIG 10B; p>0.05). Moreover, quantification of TUNEL-positive tumor cells in the same areas of the tumor cross-sections, revealed low levels of tumor cell apoptosis following adenovirus administration and local tumor irradiation (Table 1).

Table 1 depicts the expression of ASMase in tumor endothelium of asmase^{-/-}mice does not lead to radiation-induced tumor cell apoptosis. 1x10¹⁰ PFU of Ad5Empty or Ad5HEPPE-3x(ASM) was intravenously administered to asmase^{-/-} mice bearing MCA/129 fibrosarcoma. Five days post administration of virus, tumors were locally irradiated with 15 Gy or left untreated. Tumor samples were obtained before or 4, 6, 8 and 10 hours following irradiation, fixed in paraformaldehyde, and embedded in paraffin blocks. Tissue sections were stained with TUNEL antibody to visualize apoptotic nuclei and hematoxylin and eosin to visualize tumor cells. Data (mean ± SE) represent TUNEL-positive tumor cells quantified from five 400x magnification fields from an experiment employing two animals per group.

**Table 1. Expression of ASMase in tumor endothelium of asmase^{-/-} mice does not lead to radiation-induced tumor cell apoptosis**

| Vector | IR Dose | Time After IR | % Epithelial Apoptosis per 400x field |
|---|---|---|---|
| Ad5Empty | 0 Gy | N/A | 1.2 ± 0.1% |
| Ad5HEPPE-3x(ASM) | | | 1.9 ± 0.3% |
| Ad5Empty | 15 Gy | 4 hr | 1.7 ± 0.2% |
| Ad5HEPPE-3x(ASM) | | | 1.7 ± 0.2% |
| Ad5Empty | 15 Gy | 6 hr | 2.2 ± 0.3% |
| Ad5HEPPE-3x(ASM) | | | 2.8 ± 0.3% |
| Ad5Empty | 15 Gy | 8 hr | 3.1 ± 0.2% |
| Ad5HEPPE-3x(ASM) | | | 2.9 ± 0.4% |
| Ad5Empty | 15 Gy | 10 hr | 3.5 ± 0.4% |
| Ad5HEPPE-3x(ASM) | | | 3.2 ± 0.2% |

Following genetic upregulation of ASMase, apoptosis was observed in 1.9% ± 0.3% of tumor cells, and radiation had no affect on the these levels, as 1.7±0.2%, 2.8 ± 0.3%, 2.9 ± 0.4% and 3.2 ± 0.2% of tumor apoptotic cells were observed in the total population 4, 6, 8 and 10 hours after 15 Gy, respectively. Similarly, no significant difference in tumor cell apoptosis was observed in tumors treated with Ad5HEPPE-3x(ASM) and Ad5Empty (Table 1), showing that genetic upregulation of ASMase does not mediate its curative effects through tumor cell apoptosis. Collectively, these data show that reinstitution of ASMase expression in ASMase-deficient tumor endothelium restores endothelial sensitivity to radiation-induced apoptosis, which reengages the vascular component of tumor response to radiation, leading to complete tumor regression.

As Ad5HEPPE-3x(ASM) led to expression of the human ASMase in murine tumors, the fact that tumor response to radiation was indeed mediated by ASMase restoration and not by an unexpected immune reaction was studied. In order to address this possible concern, the radiation response of MCA/129 fibrosarcoma implanted into asmase^{-/-}animals harboring the SCID mutation was investigated. These mice, which display a phenotype devoid of mature host B and T lymphocytes, enable the study of the impact of irradiation in a setting with little potential for interference from immune function. Immunocompromised SCID-asmase^{-/-} mice were implanted with MCA/129 fibrosarcoma and subsequently infected with Ad5HEPPE-3x(ASM), and the tumor response to radiation was studied. It was hypothesized that if the radiosensitizing effect observed in previous studies was in fact mediated by an immune reaction, rather than by ASMase restoration, it would not be possible to observe tumor cure or growth delay in immunocompromised animals. As shown in FIG. 11, intravenous administration of Ad5HEPPE-3x(ASM) and restoration of ASMase expression in endothelium of MCA/129 fibrosarcoma implanted into SCID-asmase^{-/-}mice lead to radiosensitization similar to that observed in non-SCID-asmase^{-/-} mice infected with Ad5HEPPE-3x(ASM). Further, the radiation response of MCA/129 fibrosarcoma in SCID-asmase^{-/-} mice was restored to levels similar to SCID-asmase^{+/+} mice. While no tumors in SCID-asmase^{-/-} mice treated with Ad5Empty exhibited a response to 17 Gy, all tumors in SCID-asmase^{-/-} mice treated with Ad5HEPPE-3x(ASM) exhibited a tumor growth delay analogous to that observed in non-virus-treated SCID-asmase^{+/+} mice (FIG. 11). Tumor growth in these mice, however, could be followed only for 6 days post radiation due to the inherent radiosensitivity of SCID mice. Within 6 days of 17 Gy irradiation, all the animals exhibited severe weight loss and loss of motility, indicative of radiation-induced GI toxicity, later confirmed by necropsy analysis (data not shown). Nevertheless, the ability of Ad5HEPPE-3x(ASM) to restore the radiosensitivity of tumors in implanted in immunocompromised SCID-asmase^{-/-} mice compared to that observed in tumors implanted in wild-type littermates confirms that the Ad5HEPPE-3x(ASM) effect is mediated by ASMase expression and not by a systemic immune response to the ASMase gene.

### Example 8

### Overexpression of ASMase in wild type tumor endothelium radiosensitizes MCA/129 fibrosarcoma

Previous *in vitro* studies showed that overexpression of ASMase in BAEC leads to radiosensitization of cells with a dose modifying factor of 1.35. In order to determine whether radiosensitization can also be achieved in wild-type neovasculature *in vivo,* Ad5HEPPE-3x(ASM) was administered to MCA/129 fibrosarcoma-bearing mice and the impact of ASMase genetic upregulation on the tumor response to single-dose radiotherapy was studied.

FIG. 12 shows that exposure of MCA/129 fibrosarcoma-bearing SV129/C57^{asm+/+JAX} mice to a single dose of 14.5 Gy following infection with Ad5Empty had no significant effect on tumor growth (p>0.1). Conversely, genetic upregulation of ASMase in tumor vasculature through intravenous administration of Ad5HEPPE-3x(ASM) to MCA/129 fibrosarcoma-bearing SV129/C57^{asm+/+JAX} mice significantly increased tumor response to radiation. Exposure to 14.5 Gy resulted in local complete regression in 3 out of 10 tumors (30%), maintained for at least 90 days. The remaining 7 mice experienced a mean tumor growth delay of 9.7±5.0 days (FIG. 12C; p<0.005 vs. Ad5Empty and radiation controls). Escalation of the radiation dose to 17 Gy enhanced the effect of Ad5HEPPE-3x(ASM) on the 129/MCA fibrosarcoma tumor response to radiation. Similar to the results observed with 14.5 Gy, 17 Gy single-dose radiation of MCA/129 fibrosarcoma-bearing SV129/C57^{asm+/+JAX} mice infected with Ad5Empty had no effect on tumor growth (p>0.1) (Table 2).

**Table 2. Overexpression of ASMase via Ad5HEPPE-3x(ASM) radiosensitizes tumors**

| Tumor | Radiation Dose | Tumor Regression Rate (%) | |
|---|---|---|---|
| | | Ad5Empty | Ad5HEPPE-3x(ASM) |
| MCA/129 fibrosarcoma | 14.5 Gy | 0 | 30 |
| MCA/129 fibrosarcoma | 17 Gy | 0 | 60 |
| MCA/129 fibrosarcoma | 20 Gy | 20 | 80 |
| B16F1 melanoma | 34 Gy | 0 | 25 |
| B16F1 melanoma | 41 Gy | 0 | 66 |

In contrast to what was seen following local tumor irradiation with 17 Gy following AdEmpty infection, genetic upregulation of ASMase in the endothelium via infection with Ad5HEPPE-3x(ASM) resulted in tumor radiosensitization. Local complete regressions were observed and maintained for 90 days in 3 out of 5 MCA/129 fibrosarcoma-bearing SV129/C57^{asm+/+JAX} mice (60%). A mean tumor growth delay of 18±5.6 days was observed in the remaining 2 mice (FIG. 13C; p<0.005 vs. Ad5Empty and radiation controls).

Escalation of radiation dose to 20 Gy resulted in local complete tumor regression in 1 out 5 MCA/129 fibrosarcoma-bearing SV129/C57^{asm+/+JAX} mice (20%) infected with Ad5Empty (FIG. 14A and 14C and Table 2). Additionally, a mean tumor growth delay of 16.7±2.4 days was observed in 2 mice, while no significant impact on tumor growth was observed in the remaining 2 animals. Genetic upregulation of ASMase via Ad5HEPPE-3x(ASM), further radiosensitized tumors to single dose radiation of 20 Gy. Local complete tumor regression was observed in 4 out of 5 mice (80%), and a tumor growth delay of 10 days was observed in the remaining animal. In contrast to restoration of ASMase expression in asmase^{-/-} mice (Sloan-Kettering colony), however, no significant effect on tumor growth in the absence of radiation was observed following genetic upregulation of ASMase in SV129/C57^{asm+/+JAX} mice (FIG. 12B, 13B and 14B; p>0.05)

Overall these data show that genetic upregulation of ASMase via Ad5HEPPE-3x(ASM) in asmase^{+/+} vasculature has a significant radiosensitizing effect on mouse tumors, but does not affect tumor growth in the absence of radiation. Since treatment of animals with Ad5HEPPE-3x (ASM) plus 14.5 Gy single-dose radiation yielded an effect analogous to that seen when animals were treated with 23 Gy single-dose radiation in the absence of ASMase overexpression (data not shown), i.e. 30% complete tumor regression rate, ASMase upregulation resulted in radiosensitization with a clinically significant dose-modifying factor of 1.58.

Furthermore, radiosensitization is dependent on adenovirus dose. A dose of 1x10¹⁰ PFU and 4 additional doses a ½ log lower than each previous dose of Ad5HEPPE-3x(ASM) was administered intravenously to MCA/129 fibrosarcoma-bearing SV129/C57^{*asm*+/+*JAX*} mice. Five days post virus administration tumors were locally irradiated with 17 Gy. Results are presented in Table 3.

**Table 3. Dose de-escalation study**

| Adenovirus | Dose | Tumor regression rate after 17 Gy (%) |
|---|---|---|
| Ad5Empty | N/A | 1/5 (20%) |
| AD5HEPPE-3x(ASM) | 3x10⁸ PFU | 1/5 (20%) |
| AD5HEPPE-3x(ASM) | 1x10⁹ PFU | 1/5 (20%) |
| AD5HEPPE-3x(ASM) | 3x10⁹ PFU | 2/5 (40%) |
| AD5HEPPE-3x(ASM) | 1x10¹⁰ PFU | 3/5 (60%) |

### Example 9

### Overexpression of ASMase in tumor endothelium radiosensitizes B16F1 melanoma

FIG. 18 and Table 2 depict that overexpression of ASMase in tumor endothelium radiosensitizes B16F1 melanoma. 1x10¹⁰ PFU of Ad5Empty or Ad5HEPPE-3x(ASM) was administered intravenously to B16F1 melanoma-bearing SV129/C57^{*asm*+/+*JAX*} mice. Four days post virus administration tumors were locally irradiated with 34 (FIG. 18A) and 41 (FIG. 18B) Gy. Response of B16F1 melanoma to treatment with Ad5Empty (black lines) or Ad5HEPPE-3x(ASM) (gray lines) and IR is presented as tumor volume. N equals number of animals per group. Tumors were measured daily up to 40 days and twice weekly thereafter. Tumor regression was confirmed by local biopsy.

These data demonstrate that Ad5HEPPE-3x(ASM) not only radiosensitizes relatively radiosensitive tumors, such as MCA/129 fibrosarcoma, but also completely radioresistant tumors, such as B16F1 melanoma.

### Example 10

### Overexpression of ASMase does not radiosensitize GI microvascular endothelium

Studies using Ad5HEPPE-3x(GFP) demonstrated that target gene expression is specific for angiogenic endothelium; there was no detectable expression within the endothelium of normal tissues. In order to confirm the specificity of ASMase expression in angiogenic endothelium, whether infection with Ad5HEPPE-3x(ASM) results in radiosensitization of the vasculature within the GI tract was tested. The GI tract was chosen for these studies because it is particularly sensitive to acute radiation exposure. Whole body or total abdominal irradiation results in GI stem cell lethality and the loss of the protective barrier that separates the contents of the lumen from the circulation. This GI syndrome, which is the primary dose-limiting toxicity for radiation treatment of the GI tract, is caused by a rapid wave of radiation-induced microvascular endothelial apoptosis within the lamina propria that cooperates with direct damage to stem cells located within the crypts of Lieberkuhn at the base of each villus. The microcolony, or crypt survival, assay directly quantifies dose-dependent lethality of the crypt stem cell compartment and is predictive of eventual animal demise from the GI syndrome. SV129/C57/BL/6 mice were subjected to 8-14 Gy total body irradiation (TBI) five days following infection with Ad5HEPPE-3x(ASM). The proximal jejunum was harvested 3.5 days following irradiation. As shown in FIG. 15, significant radiosensitization of the GI tract was not observed. Specifically, 91.6%, 59.2%, 30.2% and 6.9% crypt survival was observed in mice infected with empty vector following 8, 10, 12 and 14 Gy TBI, respectively, whereas mice infected with Ad5HEPPE-3x(ASM) displayed 93.4%, 34.6%, 23.4% and 4.1% crypt survival, respectively. Analysis of these data revealed 10% crypt survival at doses of 13.7 and 13.1 Gy, respectively, for mice infected with Ad5Empty or Ad5HEPPE-3x(ASM), resulting in a dose-modifying factor of 1.05 ± 0.29.

These data demonstrate that genetic upregulation of ASMase via Ad5HEPPE-3x(ASM) specifically affects only tissues with angiogenic vasculature, such as that of tumors, radiosensitizing angiogenic endothelium but sparing endothelium within other radiation-sensitive organs, increasing the effectiveness of radiotherapy without incurring unwanted normal tissue toxicity.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An expression vector comprising a recombinant DNA construct comprising a region coding for a functional secretory ASMase linked to transcriptional regulatory sequences that confer tissue-specific expression of said secretory ASMase, **characterised in that** said expression vector Is an adenovirus vector, wherein the transcriptional regulatory sequences are specific for tumor endothelium or the angiogenic endothelium of tumors.

2. The expression vector of claim 1, wherein the transcriptional regulatory sequences are specific for the angiogenic endothelium of tumors.

3. The expression vector of claim 2, wherein said angiogenic endothellum-specific transcriptional regulate sequences are selected from the group consisting of promoters and enhancers.

4. The expression vector of claim 3, wherein said promoter is pre-proendothelln-1 promoter or modifications thereof, such as the PPE-1 (x3) promoter.

5. The expression vector of claim 3, wherein said enhancer is HIF2α-Ets-1 enhancer.

6. The expression vector of Claim 1 for use in the treatment of cancer wherein said construct increased ASMase levels In tumor endothelium and thereby Increases radiation-Induced damage to a tumor without Increasing radiation-induced side effects.

7. The expression vector for use according to Claim 6, wherein said cancer Is a solid tumor.

8. The expression vector for use according to Claim 6, where the increase in radiation-induced damage to cancer without an increase in radiation-induced side effects is achieved by sensitizing the tumor to radiation.

9. The expression vector for use according to Claim 6, where the increase in radiation-Induced damage to cancer without an Increase in radiation-induced side effects Is achieved by sensitizing the angiogenic epithelium of the tumor to radiation.

10. The expression vector for use according to Claim 6, wherein secretory ASMase levels are Increased specifically In tumor endothelium through the administration of said construct.

11. The expression vector for use according to Claim 6, wherein ceramide levels are increased specifically in tumor endothelium through the administration of said gene therapy construct.

## Patentansprüche

1. Expressionsvektor, umfassend ein rekombinantes DNA-Konstrukt, das eine Regionscodierung für eine funktionelle sekretorische ASMase umfasst, die mit transkriptionsregulatorischen Sequenzen verbunden ist, die eine gewebespezifische Expression der sekretorischen ASMase übertragen, **dadurch gekennzeichnet, dass** der Expressionsvektor ein Adenovirusvektor ist, wobei die transkriptionsregulatorischen Sequenzen für das Tumorendothel oder das angiogene Endothel von Tumoren spezifisch sind.

2. Expressionsvektor nach Anspruch 1, wobei die transkriptionsregulatorischen Sequenzen für das angiogene Endothel von Tumoren spezifisch sind.

3. Expressionsvektor nach Anspruch 2, wobei die angiogenen endothelspezifischen transkriptionsregulatorischen Sequenzen ausgewählt sind aus der Gruppe, bestehend aus Promotoren und Enhancern.

4. Expressionsvektor nach Anspruch 3, wobei der Promotor ein Präproendothelin-1-Promotor oder Modifikationen davon, etwa der PPE-1 (3x)-Promotor, ist.

5. Expressionsvektor nach Anspruch 3, wobei der Enhancer ein HIF2a-Ets-1-Enhancer ist.

6. Expressionsvektor nach Anspruch 1 zur Verwendung in der Behandlung von Krebs, wobei das Konstrukt ASMase-Werte im Tumorendothel erhöhte und dadurch eine strahlungsinduzierte Schädigung eines Tumors ohne Zunahme der strahlungsinduzierten Nebenwirkungen erhöht.

7. Expressionsvektor zur Verwendung nach Anspruch 6, wobei der Krebs ein solider Tumor ist.

8. Expressionsvektor zur Verwendung nach Anspruch 6, wobei die Zunahme der strahlungsinduzierten Schädigung von Krebs ohne Zunahme von strahlungsinduzierten Nebenwirkungen erreicht wird, indem der Tumor gegenüber Strahlung sensibilisiert wird.

9. Expressionsvektor zur Verwendung nach Anspruch 6, wobei die Zunahme der strahlungsinduzierten Schädigung von Krebs ohne Zunahme von strahlungsinduzierten Nebenwirkungen erreicht wird, indem das angiogene Epithel des Tumors gegenüber Strahlung sensibilisiert wird.

10. Expressionsvektor zur Verwendung nach Anspruch 6, wobei sekretorische ASMase-Werte durch die Verabreichung des Konstrukts insbesondere im Tumorendothel erhöht werden.

11. Expressionsvektor zur Verwendung nach Anspruch 6, wobei Ceramidwerte durch die Verabreichung des Gentherapie-Konstrukts insbesondere im Tumorendothel erhöht werden.

## Revendications

1. Vecteur d'expression comprenant une construction d'ADN recombiné comprenant une région codant pour une ASMase sécrétoire fonctionnelle liée à des séquences régulatrices de la transcription qui confèrent une expression tissu-spécifique de ladite ASMase sécrétoire, **caractérisé en ce que** ledit vecteur d'expression est un vecteur adénoviral, les séquences régulatrices de la transcription étant spécifiques pour l'endothélium tumoral ou l'endothélium angiogénique de tumeurs.

2. Vecteur d'expression selon la revendication 1, les séquences régulatrices de la transcription étant spécifiques pour l'endothélium angiogénique de tumeurs.

3. Vecteur d'expression selon la revendication 2, lesdites séquences régulatrices de la transcription spécifiques pour l'endothélium angiogénique étant choisies dans le groupe constitué par les promoteurs et les amplificateurs.

4. Vecteur d'expression selon la revendication 3, ledit promoteur étant le promoteur pré-proendothéline-1 ou des modifications de celui-ci, telles que le promoteur PPE-1(3x).

5. Vecteur d'expression selon la revendication 3, ledit amplificateur étant l'amplificateur HIF2α-Ets-1.

6. Vecteur d'expression selon la revendication 1 destiné à être utilisé dans le traitement d'un cancer, ladite construction augmentant les taux d'ASMase dans l'endothélium tumoral et de cette manière augmentant l'endommagement provoqué par un rayonnement sur une tumeur sans augmenter les effets secondaires provoqués par le rayonnement.

7. Vecteur d'expression destiné à être utilisé selon la revendication 6, ledit cancer étant une tumeur solide.

8. Vecteur d'expression destiné à être utilisé selon la revendication 6, l'augmentation de l'endommagement provoqué par un rayonnement sur un cancer sans une augmentation d'effets secondaires provoqués par le rayonnement étant réalisée par sensibilisation de la tumeur au rayonnement.

9. Vecteur d'expression destiné à être utilisé selon la revendication 6, l'augmentation de l'endommagement provoqué par un rayonnement sur un cancer sans une augmentation d'effets secondaires provoqués par le rayonnement étant réalisée par sensibilisation de l'épithélium angiogénique de la tumeur au rayonnement.

10. Vecteur d'expression destiné à être utilisé selon la revendication 6, les taux d'ASMase sécrétoire étant accrus spécifiquement dans l'endothélium tumoral grâce à l'administration de ladite construction.

11. Vecteur d'expression destiné à être utilisé selon la revendication 6, les taux de céramides étant accrus spécifiquement dans l'endothélium tumoral grâce à l'administration de ladite construction de thérapie génique.
